# EUROPÄISCHE PATENTSCHRIFT

(11) **EP 1 581 485 B1**
(45) Veröffentlichungstag und Bekanntmachung des Hinweises auf die Patenterteilung: **21.03.2007**
(21) Anmeldenummer: 03799498.5
(22) Anmeldetag: 22.12.2003
(51) Int. Cl.: C07C 303/06, C11D 1/22, C11D 11/04

(54) **VERFAHREN ZUR HERSTELLUNG VON ALKYLARYLSULFONATEN MITTELS MODIFIZIERTER, DIMERISIERTER OLEFINE**
METHODS FOR PRODUCING ALKYLARYL SULFONATES BY USING MODIFIED DIMERIZED OLEFINS
PROCEDES POUR PRODUIRE DES SULFONATES D'ALKYLARYLE AU MOYEN D'OLEFINES DIMERISEES MODIFIEES

(30) Priorität: 23.12.2002 DE 10261481
(43) Veröffentlichungstag der Anmeldung: 05.10.2005
(73) Patentinhaber: BASF Aktiengesellschaft, 67056 Ludwigshafen (DE)
(72) Erfinder: NARBESHUBER, Thomas, 68165 Mannheim (DE); STEINBRENNER, Ulrich, 67435 Neustadt (DE); WIEBELHAUS, Dag, 67435 Neustadt (DE); BOTTKE, Nils, 68165 Mannheim (DE)
(74) Vertreter: Isenbruck, Günter
(86) Internationale Anmeldenummer: PCT/EP2003/014712
(87) Internationale Veröffentlichungsnummer: WO 2004/058692

(56) Entgegenhaltungen:
- WO-A-02/14266

## Beschreibung

Die vorliegende Erfindung betrifft Verfahren zur Herstellung von Alkylarylsulfonaten, nach dem Verfahren erhältliche Alkylarylsulfonate sowie in dem Verfahren als Zwischenprodukt erhältliche Alkylaryle, die Verwendung der Alkylarylsulfonate als Tenside, vorzugsweise in Wasch- und Reinigungsmitteln, und diese enthaltende Wasch- und Reinigungsmittel.

Alkylbenzolsulfonate (ABS) werden seit langer Zeit als Tenside in Wasch- und Reinigungsmitteln eingesetzt. Nachdem zunächst derartige Tenside auf Basis von Tetrapropylen eingesetzt wurden, die jedoch schlecht biologisch abbaubar waren, wurden in der Folgezeit möglichst lineare Alkylbenzolsulfonate (LAS) hergestellt und verwendet. Lineare Alkylbenzolsulfonate weisen jedoch nicht für alle Anwendungsbereiche ausreichende Eigenschaftsprofile auf.

So wäre es zum Beispiel vorteilhaft, ihre Kaltwascheigenschaften oder ihre Eigenschaften in hartem Wasser zu verbessern. Ebenso wünschenswert ist die leichte Formulierbarkeit, die sich aus der Viskosität der Sulfonate und deren Löslichkeit ergibt. Diese verbesserten Eigenschaften werden durch geringfügig verzweigte Verbindungen bzw. Mischungen von geringfügig verzweigten Verbindungen mit linearen Verbindungen erreicht, wobei man jedoch das richtige Maß an Verzweigung und/oder das richtige Maß an Mischung erzielen muß. Zu starke Verzweigungen benachteiligen die biologische Abbaubarkeit der Produkte. Zu lineare Produkte beeinflussen die Viskosität und die Löslichkeit der Sulfonate negativ.

Darüber hinaus spielt der Anteil an terminalen Phenylalkanen (2-Phenylalkane und 3-Phenylalkane) zu internen Phenylalkanen (4-, 5-, 6- etc. Phenylalkane) eine Rolle für die Produkteigenschaften. Ein 2-Phenylanteil von etwa 30 % und ein 2- und 3-Phenylanteil von etwa 50 % können hinsichtlich der Produktqualität (Löslichkeit, Viskosität, Wascheigenschaften) vorteilhaft sein.

Tenside mit zu hohen 2- und 3-Phenylgehalten können den wesentlichen Nachteil aufweisen, dass die Verarbeitbarkeit der Produkte durch einen starken Anstieg der Viskosität der Sulfonate leidet.

Darüber hinaus kann sich ein nicht-optimales Löslichkeitsverhalten ergeben. So ist z.B. der Krafft-Punkt einer Lösung von LAS mit sehr hohen oder sehr niedrigen 2- und 3-Phenylanteilen um bis zu 10-20 °C höher als bei optimaler Wahl des 2- und 3-Phenylanteils.

Das erfindungsgemäße Verfahren bietet den wesentlichen Vorteil, daß durch die Kombination von Metathese und Dimerisierung ein einzigartiges Olefingemisch erhalten wird, welches nach Alkylierung eines Aromaten, Sulfonierung und Neutralisation ein Tensid liefert, das sich durch seine Kombination von hervorragenden Anwendungseigenschaften (Löslichkeit, Viskosität, Stabilität gegen Wasserhärte, Wascheigenschaften, biologischer Abbaubarkeit) auszeichnet. Hinsichtlich der biologischen Abbaubarkeit von Alkylarylsulfonaten sind Verbindungen, die weniger stark an Klärschlamm adsorbiert werden als herkömmliches LAS, besonders vorteilhaft.

Daher sind zu einem gewissen Grad verzweigte Alkylbenzolsulfonate entwickelt worden.

WO 99/05241 betrifft Reinigungsmittel, die verzweigte Alkylarylsulfonate als Tenside enthalten. Die Alkylarylsulfonate werden durch Dimerisierung von Olefinen zu Vinylidinolefinen und nachfolgende Alkylierung von Benzol an einem formselektiven Katalysator wie MOR oder BEA erhalten. Darauf folgt eine Sulfonierung.

WO 02/44114 betrifft ein Verfahren zur Herstellung von Alkylarylsulfonaten, bei dem nach unterschiedlichen Verfahren erhältliche einfach-verzweigte C₁₀₋₁₄-Olefine mit einem aromatischen Kohlenwasserstoff in Gegenwart von Zeolithen des Typs Faujasit als Alkylierungskatalysator umgesetzt werden. Die C₁₀₋₁₄-Olefine können beispielsweise durch Metathese eines C₄-Olefin-Gemisches, gefolgt von einer Dimerisierung des erhaltenen 2-Pentens und/oder 3-Hexens an einem Dimerisierungskatalysator, hergestellt werden. Alternative Verfahren sind eine Extraktion, Fischer-Tropsch-Synthese, Dimerisierung oder Isomerisierung von Olefinen.

WO 02/14266 betrifft ein Verfahren zur Herstellung von Alkylarylsulfonaten, bei dem zunächst eine Metathese eines C₄-Olefin-Gemisches zur Herstellung von 2-Penten und/oder 3-Hexen durchgeführt wird und die Produkte einer Dimerisierung unterworfen werden. Sodann erfolgt eine Alkylierung in Gegenwart eines Alkylierungskatalysators, worauf sich eine Sulfonierung und Neutralisation anschließt.

Die bislang zur Alkylierung eingesetzten Olefine weisen teilweise einen zu hohen oder zu niedrigen Verweigungsgrad auf bzw. ergeben ein nicht optimales Verhältnis terminaler zu interner Phenylalkane. Zum anderen Teil werden sie aus teuren Ausgangsstoffen wie zum Beispiel Propen oder alpha-Olefinen hergestellt, und teilweise beträgt der Anteil der für die Tensidherstellung interessanten Olefinfraktionen nur etwa 20 %. Dies führt zu teuren Aufarbeitungsschritten. Die letztgenannten Verfahren führen nicht in allen Fällen zu Produkten, die ein gewünschtes Eigenschaftsspektrum zeigen.

Aufgabe der vorliegenden Erfindung ist die Bereitstellung eines Verfahrens zur Herstellung von Alkylarylsulfonaten, die zumindest teilweise verzweigt sind und damit für den Einsatz in Wasch- und Reinigungsmitteln gegenüber den bekannten Verbindungen vorteilhafte Eigenschaften aufweisen. Sie sollen insbesondere ein geeignetes Eigenschaftsprofil aus biologischer Abbaubarkeit, Unempfindlichkeit gegen Wasserhärte, Löslichkeit und Viskosität bei der Herstellung und beim Einsatz aufweisen. Zudem sollen die Alkylarylsulfonate kostengünstig herstellbar sein.

Die Aufgabe wird erfindungsgemäß gelöst durch ein Verfahren zur Herstellung von Alkylarylsulfonaten durch
a) Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen,
b) Dimerisierung des in Stufe a) erhaltenen 2-Pentens und/oder 3-Hexens in Gegenwart eines Dimerisierungskatalysators zu einem C₁₀₋₁₂-Olefine enthaltenden Gemisch, Abtrennung der C₁₀₋₁₂-Olefine und Abtrennung von 5 bis 30 Gew.-%, bezogen auf die abgetrennten C₁₀₋₁₂-Olefine, an Leichtsieder-Bestandteilen der C₁₀₋₁₂-Olefine,
c) Umsetzung der in Stufe b) erhaltenen C₁₀₋₁₂-Olefin-Gemische mit einem aromatischen Kohlenwasserstoff in Gegenwart eines Alkylierungskatalysators zur Bildung von alkylaromatischen Verbindungen, wobei vor der Umsetzung zusätzlich 0 bis 60 Gew.-%, bevorzugt 0 bis 40 Gew.-%, bezogen auf die in Stufe b) erhaltenen C₁₀₋₁₂-Olefin-Gemische, an linearen Olefinen zugesetzt werden können,
d) Sulfonierung der in Stufe c) erhaltenen alkylaromatischen Verbindungen und Neutralisation zu Alkylarylsulfonaten, wobei vor der Sulfonierung zusätzlich 0 bis 60 Gew.-%, bevorzugt 0 bis 50 Gew.-% bezogen auf die in Stufe c) erhaltenen alkylaromatischen Verbindungen an linearen Alkylbenzolen zugesetzt werden können, sofern keine Zumischung in Stufe c) erfolgt ist,
e) gegebenenfalls Abmischen der in Stufe d) erhaltenen Alkylarylsulfonate mit 0 bis 60 Gew.-%, bevorzugt 0 bis 30 Gew.-% bezogen auf die in Stufe d) erhaltenen Alkylarylsulfonate, an linearen Alkylarylsulfonaten, sofern keine Zumischungen in Stufen c) und d) erfolgt sind,
sowie durch ein Verfahren zur Herstellung von Alkylarylsulfonaten durch
a) Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen,
b) Dimerisierung des in Stufe a) erhaltenen 2-Pentens und/oder 3-Hexens in Gegenwart eines Dimerisierungskatalysators zu einem C₁₀₋₁₂-Olefine enthaltenden Gemisch und gegebenenfalls Abtrennung der C₁₀₋₁₂-Olefine,
c) Umsetzung der in Stufe b) erhaltenen C₁₀₋₁₂-Olefin-Gemische mit einem aromatischen Kohlenwasserstoff in Gegenwart eines Alkylierungs-katalysators zur Bildung von alkylaromatischen Verbindungen, wobei vor der Umsetzung zusätzliche lineare Olefine zugesetzt werden können,
d) Sulfonierung der in Stufe c) erhaltenen alkylaromatischen Verbindungen und Neutralisation zu Alkylarylsulfonaten, wobei vor der Sulfonierung zusätzlich lineare Alkylbenzole zugesetzt werden können,
e) gegebenenfalls Abmischen der in Stufe d) erhaltenen Alkylarylsulfonate mit linearen Alkylarylsulfonaten,
wobei in mindestens einer der Stufen c), d) und e) 5 bis 60 Gew.-%, jeweils bezogen auf die in der vorherigen Stufe erhaltenen Gemische, der linearen Verbindungen zugesetzt werden und die Summe der Zusetzungen nicht mehr als 80 Gew.-%, bevorzugt nicht mehr als 60 Gew.-%, besonders bevorzugt nicht mehr als 50 Gew.% beträgt.
Die Kombination einer Metathese von C₄-Olefinen mit einer nachfolgenden Dimerisierung und Alkylierung von aromatischen Kohlenwasserstoffen erlaubt unter den genannten Bedingungen die Verwendung preisgünstiger Ausgangsstoffe und von Herstellungsverfahren, welche die gewünschten Produkte in hohen Ausbeuten zugänglich machen.

Es wurde erfindungsgemäß gefunden, dass durch Metathese von C₄-Olefinen Produkte erhalten werden, die sich zu leicht verzweigten C₁₀₋₁₂-Olefin-Gemischen dimerisieren lassen. Diese Gemische lassen sich durch Einstellen des gewünschten Verzweigungsgrades, zum Beispiel durch selektive Dimerisierung oder Abtrennung eines Leichtsiederteils und/oder Zusatz linearer Olefine vorteilhaft bei der Alkylierung von aromatischen Kohlenwasserstoffen einsetzen, wobei Produkte erhalten werden, die nach Sulfonierung und Neutralisation Tenside ergeben, die überragende Eigenschaften, insbesondere hinsichtlich der Empfindlichkeit gegen Härte bildende Ionen, der Löslichkeit der Sulfonate, der Viskosität der Sulfonate und ihrer Wascheigenschaften aufweisen. Darüber hinaus ist das vorliegende Verfahren äußerst kostengünstig, da die Produktströme so flexibel gestaltet werden können, dass keine Nebenprodukte anfallen. Ausgehend von einem C₄-Strom werden durch die erfindungsgemäße Metathese lineare, interne Olefine hergestellt, die sodann über den Dimerisierungsschritt in verzweigte Olefine überführt werden.

Stufe a) des erfindungsgemäßen Verfahrens ist die Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen. Die Metathese kann beispielsweise wie in WO 00/39058 oder DE-A-100 13 253 beschrieben, durchgeführt werden.

Die Olefinmetathese (Disproportionierung) beschreibt in ihrer einfachsten Form die reversible, metallkatalysierte Umalkylidenierung von Olefinen durch Bruch oder Neuformierung von C=C-Doppelbindungen gemäß nachfolgender Gleichung:

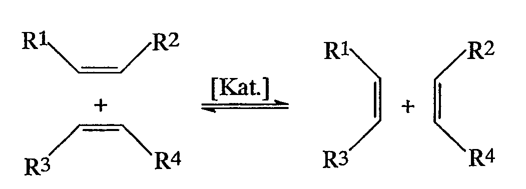

Im speziellen Fall der Metathese von acyclischen Olefinen unterscheidet man zwischen Selbstmetathese, bei der ein Olefin in ein Gemisch zweier Olefine unterschiedlicher molarer Masse übergeht (beispielsweise: Propen → Ethen + 2-Buten), und Kreuz- oder Co-Metathese, die eine Reaktion zweier unterschiedlicher Olefine beschreibt (Propen + 1-Buten → Ethen + 2-Penten). Ist einer der Reaktionspartner Ethen, so spricht man im allgemeinen von einer Ethenolyse.

Als Metathesekatalysatoren eignen sich prinzipiell homogene und heterogene Übergangsmetall-Verbindungen, insbesondere die der VI. bis VIII.-Nebengruppe des Periodensystems der Elemente sowie homogene und heterogene Katalysatorsysteme, in denen diese Verbindungen enthalten sind.

Unterschiedliche Metathese-Verfahren, die von C₄-Strömen ausgehen, sind erfindungsgemäß einsetzbar.

Die DE-A-199 32 060 betrifft ein Verfahren zur Herstellung von C₅-/C₆-Olefinen durch Umsetzung eines Ausgangsstroms, der 1-Buten, 2-Buten und Isobuten enthält, zu einem Gemisch aus C₂₋₆-Olefinen. Dabei wird aus Butenen insbesondere Propen gewonnen. Zusätzlich werden Hexen und Methylpenten als Produkte ausgeschleust. In der Metathese wird kein Ethen zudosiert. Gegebenenfalls wird in der Metathese gebildetes Ethen in den Reaktor zurückgeführt.

Ein bevorzugtes Verfahren zur Herstellung von gegebenenfalls Propen und Hexen aus einem, olefinische C₄-Kohlenwasserstoffe enthaltenden Raffinat-II-Ausgangsstrom ist dadurch gekennzeichnet, dass
a) in Gegenwart eines Metathesekatalysators, der mindestens eine Verbindung eines Metalls der VI.b, VII.b oder VIII. Nebengruppe des Periodensystems der Elemente enthält, eine Metathesereaktion durchgeführt wird, im Rahmen derer im Eingangsstrom enthaltene Butene mit Ethen zu einem Ethen, Propen, Butene, 2-Penten, 3-Hexen und Butane enthaltenden Gemisch umgesetzt werden, wobei bezogen auf die Butene bis 0,6 Moläquivalente Ethen eingesetzt werden können,
b) der so erhaltene Austragsstrom zunächst destillativ getrennt wird in gegebenenfalls eine C₂-C₃-Olefine enthaltende Leichtsiederfraktion **A** sowie in eine C₄-C₆-Olefine und Butane enthaltende Schwersiederfraktion,
c) die aus b) gegebenenfalls erhaltene Leichtsiederfraktion A anschließend destillativ in eine Ethen enthaltende Fraktion und eine Propen enthaltende Fraktion getrennt wird, wobei die Ethen enthaltende Fraktion in den Verfahrensschritt a) zurückgeführt wird und die Propen enthaltende Fraktion als Produkt ausgeschleust wird,
d) die aus b) erhaltene Schwersiederfraktion anschließend destillativ in eine Butene und Butane enthaltende Leichtsiederfraktion **B,** eine 2-Penten enthaltende Mittelsiederfraktion **C** und in eine 3-Hexen enthaltende Schwersiederfraktion **D** getrennt wird,
e) wobei die Fraktionen **B** und gegebenenfalls **C** komplett oder teilweise in den Verfahrensschritt a) zurückgeführt werden und die Fraktion **D** und gegebenenfalls **C** als Produkt ausgeschleust werden.

Ein alternatives bevorzugtes Verfahren zur Herstellung von C₆-Alkenen aus einem C₄-Alkene enthaltenden Kohlenwasserstoffstrom (Ausgangsstrom C₄⁼) ist dadurch gekennzeichnet, dass man
a) in einem Schritt a) den Strom C₄⁼ mit einem Metathesekatalysator, der mindestens eine Verbindung eines Metalls der VI.b, VII.b oder VIII. Nebengruppe des Periodensystems der Elemente enthält, in Kontakt bringt, wobei zumindest ein Teil der C₄-Alkene zu C₂- bis C₆-Alkenen umgesetzt wird, und den dabei gebildeten C₂-bis C₆-Alkene enthaltenden Stoffstrom (Strom C₂₋₆⁼) von dem Metathesekatalysator abtrennt,
b) in einem Schritt b) aus dem Strom C₂₋₆⁼ Ethylen destillativ entfernt und so einen C₃-bis C₆-Alkene enthaltenden Stoffstrom herstellt (Strom C₃₋₆⁼) und einen im wesentlichen aus Ethylen bestehenden Stoffstrom (Strom C₂⁼) herstellt,
c) in einem Schritt c) den Strom C₃₋₆⁼ destillativ in einen im wesentlichen aus Propylen bestehenden Stoffstrom (Strom C₃⁼), einen im wesentlichen aus C₆-Alkenen bestehenden Stoffstrom (Strom C₆⁼) und einen oder mehrere Stoffströme, ausgewählt aus folgender Gruppe: einen im wesentlichen aus C₄-Alkenen bestehenden Stoffstrom (Strom C₄⁼), einen im wesentlichen aus C₅-Alkenen bestehenden Stoffstrom (Strom C₅⁼) und einen im wesentlichen aus C₄- und C₅-Alkenen bestehenden Stoffstrom (Strom C₄₋₅⁼) auftrennt,
d) in einem Schritt d) einen oder mehrere Stoffströme bzw. Teile davon, ausgewählt aus der Gruppe Strom C₄⁼, Strom C₅⁼ und Strom C₄₋₅⁼ ganz oder teilweise zur Herstellung von Ausgangsstrom C₄⁼ verwendet (Recyclestrom), und gegebenenfalls den oder die Ströme, bzw. den oder die Teile davon, die nicht Recyclestrom sind, ausschleust.

Der Ausgangsstrom C₄⁼ wird dabei nach einem Verfahren, wie es in der EP-A 1069101 beschrieben ist, einer Metathesereaktion unterworfen.

Die Metathesereaktion gemäß Schritt a) wird dabei vorzugsweise in Gegenwart von heterogenen, nicht oder nur geringfügig isomerisierungsaktiven Metathesekatalysatoren durchgeführt, die aus der Klasse der auf anorganischen Trägern aufgebrachten Übergangsmetallverbindungen von Metallen der VI.b, VII.b oder VIII. Gruppe des Periodensystems der Elemente ausgewählt sind.

Bevorzugt wird als Metathesekatalysator Rheniumoxid auf einem Träger, vorzugsweise auf γ-Aluminiumoxid oder auf Al₂O₃/B₂O₃/SiO₂-Mischträgern eingesetzt.

Insbesondere wird als Katalysator Re₂O₇/γ-Al₂O₃ mit einem Rheniumoxidgehalt von 1 bis 20 Gew.-%,vorzugsweise 3 bis 15 Gew.-%, besonders bevorzugt 6 bis 12 Gew.-% eingesetzt.

Die Metathese wird bei Flüssigfahrweise vorzugsweise bei einer Temperatur von 0 bis 150°C, besonders bevorzugt 20 bis 80°C sowie einem Druck von 2 bis 200 bar, besonders bevorzugt 5 bis 30 bar, durchgeführt.

Wenn die Metathese in der Gasphase durchgeführt wird, beträgt die Temperatur vorzugsweise 20 bis 300°C, besonders bevorzugt 50 bis 200°C. Der Druck beträgt in diesem Fall vorzugsweise 1 bis 20 bar, besonders bevorzugt 1 bis 5 bar. Detaillierte Angabe zur Metathesereaktion finden sich wiederum in der EP-A 1069101.

Die nachfolgende Aufarbeitung des bei der Metathese gebildeten Stroms C₂₋₆⁼ erfolgt in den eingangs beschriebenen Schritten b) und c).

Bevorzugt wird dabei in Schritt c) nach den 3 folgenden alternativen Verfahren vorgegangen:
Variante 1 wird in Form von 2 Teilschritten c1) und c2) üblicherweise in zwei getrennten Kolonnen durchgeführt, indem man
   c1) in Schritt c1) den Strom C₃₋₆⁼ destillativ in einen im wesentlichen aus Propylen bestehenden Stoffstrom (Strom C₃⁼), üblicherweise als Kopfabzug, und einen im wesentlichen aus C₄-Alkenen, C₅- und C₆-Alkenen bestehenden Stoffstrom (Strom C₄₋₆⁼), üblicherweise als Bodenabzug, auftrennt und
   c2) in Schritt c2) den Strom C₄₋₆⁼ destillativ in einen im wesentlichen aus Butenen bestehenden Stoffstrom (Strom C₄⁼), üblicherweise als Kopfabzug, und einen im wesentlichen aus C₄- und C₅-Alkenen bestehenden Stoffstrom (Strom C₄₋₅⁼), üblicherweise als Kopfabzug, und einen Strom C₆⁼ auftrennt [Variante CS]
Variante 2 wird in Form von 2 Teilschritten c3) und c4) üblicherweise in zwei getrennten Kolonnen durchgeführt, indem man
   c3) in Schritt c3) den Strom C₃₋₆⁼ destillativ in einen im wesentlichen aus C₆-Alkenen bestehenden Stoffstrom (Strom C₆⁼), üblicherweise als Bodenabzug, und einen im wesentlichen aus Propen, Butenen und C₅-Alkenen bestehenden Stoffstrom (Strom C₄₋₅⁼), auftrennt, üblicherweise als Kopfabzug und
   c4) in Schritt c4) den Strom C₄₋₅⁼ destillativ in einen im wesentlichen aus Propen bestehenden Stoffstrom (Strom C₃⁼), üblicherweise als Kopfabzug, einen im wesentlichen aus C₄-Alkenen bestehenden Stoffstrom (Strom C₄⁼), üblicherweise als Seitenabzug, und einen im wesentlichen aus Butenen und C₅-Alkenen bestehenden Stoffstrom (Strom C₄₋₅⁼), üblicherweise als Bodenabzug, auftrennt. [Variante DS]
Variante 3 wird in Form von 3 Teilschritten c5) bis c7) üblicherweise in drei getrennten Kolonnen durchgeführt, indem man
   c5) in Schritt c5) den Strom C₃₋₆⁼ destillativ in einen im wesentlichen aus Propen und C₄-Alkenen bestehenden Stoffstrom (Strom C₃₋₄⁼), üblicherweise als Kopfabzug, und einen im wesentlichen aus C₅- und C₆-Alkenen bestehenden Stoffstrom (Strom C₅₋₆⁼), üblicherweise als Bodenabzug, auftrennt und
   c6) in Schritt c6) den Strom C₃₋₄⁼ destillativ in einen Strom C₃⁼, üblicherweise als Kopfabzug, und einen Strom C₄⁼, üblicherweise als Bodenabzug, auftrennt.
   c7) in Schritt c7) den Strom C₅₋₆⁼ destillativ in einen Strom C₅⁼, üblicherweise als Kopfabzug, und einen Strom C₆⁼, üblicherweise als Bodenabzug, auftrennt.

### [Variante F]

Die Trennleistung der Kolonnen wird im allgemeinen so eingestellt, dass dabei Propen und Ethylen mit einer Reinheit von mehr als 99 Gew.-% erhalten werden.

Die in den einzelnen Varianten des Schritts c) gebildeten Ströme C₄⁼, C₅⁼ und C₄₋₅⁼ werden als Recyclestrom gemäß Schritt d), wie bereits oben beschrieben, teilweise oder ganz zur Herstellung des Ausgangsstroms C₄⁼ verwendet.

Der Anteil der Stoffströme C₄⁼, C₅⁼ und C₄₋₅⁼, der als Recyclestrom verwendet wird, beträgt üblicherweise 10 bis 70%, bezogen auf die Summe an Recyclestrom und ausgeschleustem Anteil an den Stoffströmen C₄⁼, C₅⁼ und C₄₋₅⁼. Sofern im Stoffstrom C₄⁼ oder C₄₋₅⁼ neben 1- und 2-Buten sowie Isobuten auch C₄-Alkane anwesend sind, muss zumindest ein Teil der C₄-Alkane entfernt werden, um eine Anreicherung der C₄-Alkane zu vermeiden oder es kann nur ein Teil dieser Ströme als Recyclestrom verwendet werden.

Die einzelnen Ströme und Fraktionen können die genannten Verbindungen enthalten oder aus ihnen bestehen. Im Fall, dass sie aus den Strömen oder Verbindungen bestehen, ist die Gegenwart kleinerer Mengen anderer Kohlenwasserstoffe nicht ausgeschlossen.

Dabei wird in einstufiger Reaktionsführung in einer Metathesereaktion eine aus C₄-Olefinen, vorzugsweise n-Butenen und Butanen bestehende Fraktion gegebenenfalls mit variablen Mengen Ethen an einem homogenen oder vorzugsweise heterogenen Metathesekatalysator zu einem Produktgemisch aus (inerten) Butanen, nicht umgesetztem 1-Buten, 2-Buten sowie den Metatheseprodukten Ethen, Propen, 2-Penten und 3-Hexen umgesetzt. Die gewünschten Produkte 2-Penten und/oder 3-Hexen werden ausgeschleust, und die verbleibenden Produkte und nicht umgesetzten Verbindungen werden in die Metathese ganz oder teilweise zurückgeführt. Vorzugsweise werden sie möglichst vollständig zurückgeführt, wobei nur geringe Mengen ausgeschleust werden, um eine Aufpegelung zu vermeiden. Idealerweise kommt es zu keiner Aufpegelung und alle Verbindungen außer 3-Hexen werden in die Metathese zurückgeführt.

Erfindungsgemäß werden, bezogen auf die Butene im C₄-Feedstrom, bis 0,6, vorzugsweise bis 0,5 Moläquivalente Ethen eingesetzt. Damit werden im Vergleich zum Stand der Technik nur geringe Ethenmengen eingesetzt.

Zudem werden erfindungsgemäß maximal mögliche Mengen an im Reaktoraustrag enthaltenen C₄-Produkten und gegebenenfalls Cs-Produkten zurückgeführt. Dies betrifft insbesondere die Rückführung von nicht umgesetztem 1-Buten und 2-Buten sowie gegebenenfalls gebildetem 2-Penten.

Sofern im C₄-Feedstrom noch geringe Mengen an Isobuten enthalten sind, können auch geringe Mengen verzweigter Kohlenwasserstoffe gebildet werden.

Die Menge an möglicherweise zusätzlich gebildeten verzweigten C₅- und C₆-Kohlenwasserstoffen im Metatheseaustrag ist abhängig vom Isobuten-Gehalt im C₄-Feed und wird vorzugsweise möglichst gering (< 3 %) gehalten.

Um das erfindungsgemäße Verfahren in mehreren Variationen näher zu erläutern, wird die im Metathesereaktor stattfindende Umsetzung in drei wichtige Einzelreaktionen unterteilt:

### 1. Kreuzmetathese von 1-Buten mit 2-Buten

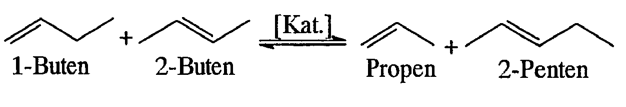

### 2. Selbstmetathese von 1-Buten

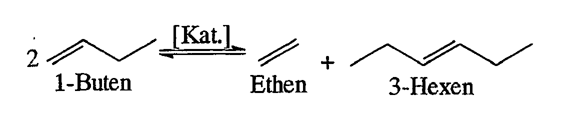

### 3. Gegebenenfalls Ethenolyse von 2-Buten

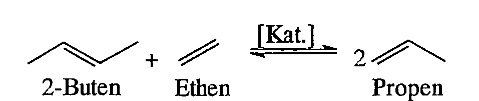

In Abhängigkeit vom jeweiligen Bedarf an den Zielprodukten Propen und 3-Hexen (die Bezeichnung 3-Hexen beinhaltet unter anderem eventuell gebildete Isomere) bzw. 2-Penten kann die äußere Massenbilanz des Verfahrens gezielt durch variablen Einsatz von Ethen und durch Verschiebung des Gleichgewichts durch Rückführung bestimmter Teilströme beeinflusst werden. So wird beispielsweise die 3-Hexenausbeute dadurch erhöht, dass durch Rückführung von 2-Penten in den Metatheseschritt die Kreuzmetathese von 1-Buten mit 2-Buten unterdrückt wird, so dass hier kein oder möglichst wenig 1-Buten verbraucht wird. Bei der dann bevorzugt ablaufenden Selbstmetathese von 1-Buten zu 3-Hexen wird zusätzlich Ethen gebildet, welches in einer Folgereaktion mit 2-Buten zum Wertprodukt Propen reagiert.

Olefingemische, die 1-Buten und 2-Buten und gegebenenfalls Isobuten enthalten, werden u.a. bei diversen Crackprozessen wie Steamcracking oder FCC-Cracking als C₄-Fraktion erhalten. Alternativ können Butengemische, wie sie bei der Dehydrierung von Butanen oder durch Dimerisierung von Ethen anfallen, eingesetzt werden. In der C₄-Fraktion enthaltene Butane verhalten sich inert. Diene, Alkine oder Enine werden vor dem erfindungsgemäßen Metatheseschritt mit gängigen Methoden wie Extraktion oder Selektivhydrierung entfernt.

Der Butengehalt der im Verfahren eingesetzten C₄-Fraktion beträgt 1 bis 100 Gew.-%, vorzugsweise 60 bis 90 Gew.-%. Der Butengehalt bezieht sich dabei auf 1-Buten, 2-Buten und Isobuten.

Vorzugsweise wird eine C₄-Fraktion eingesetzt, wie sie beim Steam- oder FCC-Cracken oder bei der Dehydrierung von Butan anfällt.

Dabei wird als C₄-Fraktion vorzugsweise Raffinat II eingesetzt, wobei der C₄-Strom vor der Metathese-Reaktion durch entsprechende Behandlung an Adsorber-Schutzbetten, bevorzugt an hochoberflächigen Aluminiumoxiden oder Molsieben von störenden Verunreinigungen befreit wird.

In Schritt d) kann die Trennung in Leichtsiederfraktion B, Mittelsiederfraktion C und Schwersiederfraktion D beispielsweise in einer Trennwandkolonne durchgeführt werden. Hierbei wird die Leichtsiederfraktion B über Kopf, die Mittelsiederfraktion C über einen Mittelaustrag und die Schwersiederfraktion D als Sumpf erhalten.

Die Metathesereaktion wird dabei vorzugsweise in Gegenwart von heterogenen, nicht oder nur geringfügig isomerisierungsaktiven Metathesekatalysatoren durchgeführt, die aus der Klasse der auf anorganischen Trägern aufgebrachten Übergangsmetallverbindungen von Metallen der VI.b, VII.b oder VIII.-Gruppe des Periodensystems der Elemente ausgewählt sind.

Bevorzugt wird als Metathesekatalysator Rheniumoxid auf einem Träger, vorzugsweise auf γ-Aluminiumoxid oder auf Al₂O₃/B₂O₃/SiO₂-Mischträgern eingesetzt.

Insbesondere wird als Katalysator Re₂O₇/γ-Al₂O₃ mit einem Rheniumoxid-Gehalt von 1 bis 20 Gew.-%, vorzugsweise 3 bis 15 Gew.-%, besonders bevorzugt 6 bis 12 Gew.-% eingesetzt.

Die Metathese wird bei Flüssigfahrweise vorzugsweise bei einer Temperatur von 0 bis 150°C, besonders bevorzugt 20 bis 110°C sowie einem Druck von 2 bis 200 bar, besonders bevorzugt 5 bis 40 bar, durchgeführt.

Wenn die Metathese in der Gasphase durchgeführt wird, beträgt die Temperatur vorzugsweise 20 bis 300°C, besonders bevorzugt 50 bis 200°C. Der Druck beträgt in diesem Fall vorzugsweise 1 bis 20 bar, besonders bevorzugt 1 bis 5 bar.

Die Herstellung von C₅/C₆-Olefinen und gegebenenfalls Propen aus Steamcracker- oder Raffinerie-C₄-Strömen kann die Teilschritte (1) bis (4) umfassen:
(1) Abtrennung von Butadien und acetylenischen Verbindungen durch gegebenenfalls Extraktion von Butadien mit einem Butadien-selektiven Lösungsmittel und nachfolgend /oder Selektivhydrierung von in Roh-C₄-Schnitt enthaltenen Butadienen und acetylenischen Verunreinigungen um einen Reaktionsaustrag zu erhalten, der n-Butene und Isobuten und im wesentlichen keine Butadiene und acetylenischen Verbindungen enthält,
(2) Abtrennung von Isobuten durch Umsetzung des in der vorstehenden Stufe erhaltenen Reaktionsaustrags mit einem Alkohol in Gegenwart eines sauren Katalysators zu einem Ether, Abtrennung des Ethers und des Alkohols, die gleichzeitig oder nach der Veretherung erfolgen kann, um einen Reaktionsaustrag zu erhalten, der n-Butene und gegebenenfalls Oxygenat-Verunreinigungen enthält, wobei gebildeter Ether ausgetragen oder zur Reingewinnung von Isobuten rückgespalten werden kann und dem Veretherungsschritt ein Destillationsschritt zur Abtrennung von Isobuten nachfolgen kann, wobei gegebenenfalls auch eingeschleuste C₃-, i-C₄- sowie C₅-Kohlenwasserstoffe destillativ im Rahmen der Aufarbeitung des Ethers abgetrennt werden können, oder Oligomerisierung oder Polymerisation von Isobuten aus dem in der vorstehenden Stufe erhaltenen Reaktionsaustrag in Gegenwart eines sauren Katalysators, dessen Säurestärke zur selektiven Abtrennung von Isobuten als Oligo- oder Polyisobuten geeignet ist, um einen Strom zu erhalten, der 0 bis 15 % Rest-Isobuten aufweist,
(3) Abtrennen der Oxygenat-Verunreinigungen des Austrags der vorstehenden Schritte an entsprechend ausgewählten Adsorbermaterialien,
(4) Metathesereaktion des so erhaltenen Raffinats II-Stromes wie beschrieben.

Vorzugsweise wird der Teilschritt Selektivhydrierung von in Roh-C₄-Schnitt enthaltenen Butadien und acetylenischen Verunreinigungen zweistufig durchgeführt durch Inkontaktbringen des Roh-C₄-Schnittes in flüssiger Phase mit einem Katalysator, der mindestens ein Metall, ausgewählt aus der Gruppe Nickel, Palladium und Platin, auf einem Träger enthält, vorzugsweise Palladium auf Aluminiumoxid, bei einer Temperatur von 20 bis 200°C, einem Druck von 1 bis 50 bar, einer Volumengeschwindigkeit von 0,5 bis 30 m³ Frischfeed pro m³ Katalysator pro Stunde und einem Verhältnis von Recycle zu Zustrom von 0 bis 30 mit einem Molverhältnis von Wasserstoff zu Diolefinen von 0,5 bis 50, um einen Reaktionsaustrag zu erhalten, in welchem neben Isobuten die n-Butene 1-Buten und 2-Buten in einem Molverhältnis von 2:1 bis 1:10, vorzugsweise von 2:1 bis 1:3, vorliegen und im wesentlichen keine Diolefine und acetylenischen Verbindungen enthalten sind. Für einen maximalen Hexenaustrag liegt vorzugsweise 1-Buten im Überschuss vor, für eine hohe Propenausbeute liegt vorzugsweise 2-Buten im Überschuss vor. Das bedeutet, dass das gesamte Molverhältnis im ersten Fall 2:1 bis 1:1 und im zweiten Fall 1:1 bis 1:3 betragen kann.

Vorzugsweise wird der Teilschritt Butadien-Extraktion aus Roh-C₄-Schnitt mit einem Butadien-selektiven Lösungsmittel durchgeführt, ausgewählt aus der Klasse polaraprotischer Lösungsmittel, wie Aceton, Furfural, Acetonitril, Dimethylacetamid, Dimethylformamid und N-Methylpyrrolidon, um einen Reaktionsaustrag zu erhalten, in welchem nach anschließend durchgeführter Selektivhydrierung/Isomerisierung die n-Butene 1-Buten und 2-Buten in einem Molverhältnis 2:1 bis 1:10, vorzugsweise von 2:1 bis 1:3, vorliegen.
Vorzugsweise wird der Teilschritt Isobuten-Veretherung in einer dreistufigen Reaktorkaskade mit Methanol oder Isobutanol, vorzugsweise Isobutanol in Gegenwart eines sauren Ionentauschers durchgeführt, in der geflutete Festbettkatalysatoren von oben nach unten durchströmt werden, wobei die Reaktor-Eingangstemperatur 0 bis 60°C, vorzugsweise 10 bis 50°C, die Ausgangstemperatur 25 bis 85°C, vorzugsweise 35 bis 75°C, der Druck 2 bis 50 bar, vorzugsweise 3 bis 20 bar und das Verhältnis von Isobutanol zu Isobuten 0,8 bis 2,0, vorzugsweise 1,0 bis 1,5 beträgt sowie der Gesamtumsatz dem Gleichgewichtsumsatz entspricht.

Vorzugsweise wird der Teilschritt Isobuten-Abtrennung durch Oligomerisierung oder Polymerisation von Isobuten ausgehend von dem nach den vorstehend beschriebenen Stufen Butadien-Extraktion und/oder Selektivhydrierung erhaltenen Reaktionsaustrag in Gegenwart eines Katalysators durchgeführt, der ausgewählt ist aus der Klasse homogener und heterogener Broensted- oder Lewis-Säuren, siehe DE-A-10013 253.

### Selektivhydrierung von Roh-C₄-Schnitt

Alkine, Alkinene und Alkadiene sind aufgrund ihrer Neigung zur Polymerisation oder ihrer ausgeprägten Neigung zur Komplexbildung an Übergangsmetallen in vielen technischen Synthesen unerwünschte Stoffe. Sie beeinträchtigen die bei diesen Reaktionen verwendeten Katalysatoren zum Teil sehr stark.

Der C₄-Strom eines Steamcrackers enthält einen hohen Anteil mehrfach ungesättigter Verbindungen wie 1,3-Butadien, 1-Butin (Ethylacetylen) und Butenin (Vinylacetylen). Je nach vorhandener Downstream-Verarbeitung werden die mehrfach ungesättigten Verbindungen entweder extrahiert (Butadien-Extraktion) oder selektiv hydriert. Im erstgenannten Fall beträgt der Restgehalt mehrfach ungesättigter Verbindungen typischerweise 0,05 bis 0,3 Gew.-%, im letztgenannten Fall typischerweise 0,1 bis 4,0 Gew.-%. Da die Restmengen an mehrfach ungesättigten Verbindungen ebenfalls bei der Weiterverarbeitung stören, ist eine weitere Anreicherung durch Selektivhydrierung auf Werte < 10 ppm erforderlich. Um einen möglichst hohen Wertproduktanteil an Butenen zu erhalten, ist die Überhydrierung zu Butanen so gering wie möglich zu halten.

### Alternativ: Extraktion von Butadien aus Roh-C₄-Schnitt

Das bevorzugte Verfahren zur Butadien-Isolierung basiert auf dem physikalischen Prinzip der Extraktivdestillation. Durch Zusatz selektiver organischer Lösungsmittel wird die Flüchtigkeit spezieller Komponenten eines Gemisches, in diesem Fall Butadien, erniedrigt. Diese bleiben daher mit dem Lösungsmittel im Sumpf der Destillationskolonne, während die destillativ zuvor nicht abtrennbaren Begleitsubstanzen über Kopf entfernt werden können. Als Lösungsmittel für die Extraktivdestillation kommen hauptsächlich Aceton, Furfural, Acetonitril, Dimethylacetamid, Dimethylformamid (DMF) und N-Methylpyrrolidon (NMP) zur Anwendung. Extraktivdestillationen eignen sich besonders für butadienreiche C₄-Crackschnitte mit einem relativ hohen Anteil an Alkinen, u.a. Methyl-, Ethyl- und Vinylacetylen sowie Methylallen.

Das vereinfachte Prinzip einer Lösungsmittel-Extraktion aus Roh-C₄-Schnitt kann wie folgt dargestellt werden: Der vollständig verdampfte C₄-Schnitt wird einer Extraktionskolonne am unteren Ende zugeführt. Das Lösungsmittel (DMF, NMP) fließt von oben dem Gasgemisch entgegen und belädt sich auf dem Weg nach unten mit besserlöslichem Butadien und geringen Mengen an Butenen. Am unteren Ende der Extraktionskolonne wird ein Teil des gewonnenen Rein-Butadiens zugeführt, um die Butene weitestgehend auszutreiben. Die Butene verlassen die Trennsäule am Kopf. In einer als Ausgaser bezeichneten weiteren Kolonne wird das Butadien durch Auskochen vom Lösungsmittel befreit und anschließend reindestilliert.

Üblicherweise wird der Reaktionsaustrag einer Butadien-Extraktivdestillation in die zweite Stufe einer Selektivhydrierung eingespeist, um den Butadien-Restgehalt auf Werte von < 10 ppm zu reduzieren.

Der nach Abtrennung von Butadien verbleibende C₄-Strom wird als C₄-Raffinat oder Raffinat I bezeichnet und enthält in der Hauptsache die Komponenten Isobuten, 1-Buten, 2-Butene sowie n- und Isobutane.

### Abtrennung von Isobuten aus Raffinat I

Bei der weiteren Auftrennung des C₄-Stromes wird nachfolgend vorzugsweise Isobuten isoliert, da es sich durch seine Verzweigung und seine höhere Reaktivität von den übrigen C₄-Komponenten unterscheidet. Neben der Möglichkeit einer formselektiven Molsiebtrennung, mit welcher Isobuten mit einer Reinheit von 99 % gewonnen werden kann und an den Molsiebporen adsorbierte n-Butene und Butan mittels eines höhersiedenden Kohlenwasserstoffs wieder desorbiert werden können, geschieht dies in erster Linie destillativ unter Verwendung eines sog. Deisobutenizers, mit welchem Isobuten gemeinsam mit 1-Buten und Isobuten über Kopf abgetrennt wird und 2-Butene sowie n-Butan incl. Restmengen an Iso- und 1-Buten im Sumpf verbleiben, oder extraktiv durch Umsetzung von Isobuten mit Alkoholen an sauren Ionenaustauschern. Hierzu werden vorzugsweise Methanol (→ MTBE) oder Isobutanol (IBTBE) eingesetzt. Die Herstellung von MTBE aus Methanol und Isobuten erfolgt bei 30 bis 100°C und leichtem Überdruck in der Flüssigphase an sauren Ionenaustauschern. Man arbeitet entweder in zwei Reaktoren oder in einem zweistufigen Schachtreaktor, um einen nahezu vollständigen Isobuten-Umsatz (> 99 %) zu erzielen. Die druckabhängige Azeotropbildung zwischen Methanol und MTBE erfordert zur Reindarstellung von MTBE eine mehrstufige Druckdestillation oder wird nach neuerer Technologie durch Methanol-Adsorption an Adsorberharzen erreicht. Alle anderen Komponenten der C₄-Fraktion bleiben unverändert.

Da geringe Anteile von Diolefinen und Acetylenen durch Polymerbildung eine Verkürzung der Lebensdauer des Ionenaustauschers bewirken können, werden vorzugsweise bifunktionelle PD-enthaltende Ionenaustauscher eingesetzt, bei denen in Gegenwart kleiner Mengen Wasserstoff nur Diolefine und Acetylene hydriert werden. Die Veretherung des Isobutens bleibt hiervon unbeeinflusst.

MTBE dient in erster Linie zur Octanzahl-Erhöhung von Fahrbenzin. MTBE und IBTBE können alternativ an sauren Oxiden in der Gasphase bei 150 bis 300°C zur Reingewinnung von Isobuten rückgespalten werden.

Eine weitere Möglichkeit zur Abtrennung von Isobuten aus Raffinat I besteht in der direkten Synthese von Oligo/Polyisobuten. An sauren homogenen und heterogen Katalysatoren, wie z.B. Wolframtrioxid auf Titandioxid, kann auf diese Weise bei Isobuten-Umsätzen bis 95 % ein Austragsstrom erhalten werden, der über einen Restanteil an Isobuten von maximal 5 % verfügt.

### Feedreinigung des Raffinat II-Stroms an Adsorbermaterialien

Zur Verbesserung der Standzeit der eingesetzten Katalysatoren für den nachfolgenden Metatheseschritt ist wie vorstehend beschrieben, der Einsatz einer Feed-Reinigung (guard bed) zur Abtrennung von Katalysatorgiften, wie beispielsweise Wasser, Oxygenates, Schwefel oder Schwefelverbindungen bzw. organischen Halogeniden erforderlich.

Verfahren zur Adsorption und adsorptiven Reinigung sind beispielsweise beschrieben in W. Kast, Adsorption aus der Gasphase, VCH, Weinheim (1988). Der Einsatz von zeolithischen Adsorbentien wird erläutert bei D.W. Breck, Zeolite Molecular Sieves, Wiley, New York (1974).

Die Entfernung von speziell Acetaldehyd aus C₃ bis C₁₅-Kohlenwasserstoffen in flüssiger Phase kann gemäß EP-A-0 582 901 erfolgen.

### Selektivhydrierung von Roh-C₄-Schnitt

Aus der aus einem Steamcracker oder einer Raffinerie stammenden Roh-C₄-Fraktion wird zunächst Butadien (1,2- und 1,3-Butadien) sowie im C₄-Schnitt enthaltene Alkine oder Alkenine in einem zweistufigen Verfahren selektivhydriert. Der aus der Raffinerie stammende C₄-Strom kann gemäß einer Ausführungsform auch direkt in den zweiten Schritt der Selektivhydrierung eingespeist werden.

Der erste Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich 40 bis 80°C und einem Druck von 10 bis 30 bar, einem Molverhältnis von Wasserstoff zu Butadien von 10 bis 50 und einer Volumengeschwindigkeit LHSV von bis 15 m³ Frischfeed pro m³ Katalysator pro Stunde und einem Verhältnis von Recycle zu Zustrom von 5 bis 20.

Der zweite Schritt der Hydrierung wird vorzugsweise an einem Katalysator durchgeführt, der 0,1 bis 0,5 Gew.-% Palladium auf Aluminiumoxid als Träger enthält. Die Umsetzung wird in Gas/Flüssigphase im Festbett (Rieselfahrweise) mit einem Flüssigkreislauf betrieben. Die Hydrierung erfolgt bei einer Temperatur im Bereich von 50 bis 90°C und einem Druck von 10 bis 30 bar, einem Molverhältnis von Wasserstoff zu Butadien von 1,0 bis 10 und einer Volumengeschwindigkeit LHSV von 5 bis 20 m³ Frischfeed pro m³ Katalysator pro Stunde und einem Verhältnis von Recycle zu Zustrom von 0 bis 15.

Der so erhaltene Reaktionsaustrag wird als Raffinat I bezeichnet und weist neben Isobuten 1-Buten und 2-Buten in einem Molverhältnis von 2:1 bis 1:10, vorzugsweise von 2:1 bis 1:3 auf.

### Alternativ: Abtrennung von Butadien aus Roh-C₄-Schnitt via Extraktion

Die Extraktion von Butadien aus Roh-C₄-Schnitt erfolgt unter Verwendung von N-Methylpyrrolidon.

Der Reaktionsaustrag der Extraktion wird gemäß einer Ausführungsform der Erfindung in den zweiten Schritt der vorangehend beschriebenen Selektivhydrierung eingespeist, um Restmengen Butadien zu entfernen, wobei in diesem Selektivhydrierungsschritt das gewünschte Verhältnis 1-Buten zu 2-Buten eingestellt wird.

### Abtrennung von Isobuten via Veretherung mit Alkoholen

In der Veretherungsstufe wird Isobuten mit Alkoholen, vorzugsweise mit Isobutanol, an einem sauren Katalysator, vorzugsweise an einem sauren Ionenaustauscher, zu Ether, vorzugsweise Isobutyl-tert.-butylether umgesetzt. Die Umsetzung erfolgt gemäß einer Ausführungsform der Erfindung in einer dreistufigen Reaktorkaskade, in der geflutete Festbettkatalysatoren von oben nach unten durchströmt werden. Im ersten Reaktor beträgt die Eingangstemperatur 0 bis 60°C, vorzugsweise 10 bis 50°C; die Ausgangstemperatur liegt zwischen 25 und 85°C, vorzugsweise zwischen 35 und 75°C, und der Druck beträgt 2 bis 50 bar, vorzugsweise 3 bis 20 bar. Bei einem Verhältnis von Isobutanol zu Isobuten von 0,8 bis 2,0, vorzugsweise 1,0 bis 1,5 beträgt der Umsatz zwischen 70 und 90 %.

Im zweiten Reaktor beträgt die Eingangstemperatur 0 bis 60°C, vorzugsweise 10 bis 50°C; die Ausgangstemperatur liegt zwischen 25 und 85, vorzugsweise zwischen 35 und 75°C, und der Druck beträgt 2 bis 50 bar, vorzugsweise 3 bis 20 bar. Der Gesamtumsatz über die zwei Stufen erhöht sich auf 85 bis 99 %, vorzugsweise 90 bis 97 %.

Im dritten und größten Reaktor wird bei gleicher Eingangs- und Ausgangstemperatur von 0 bis 60°C, vorzugsweise 10 bis 50°C, der Gleichgewichtsumsatz erzielt. An die Veretherung und Abtrennung des gebildeten Ethers schließt sich die Etherspaltung an: Die endotherme Reaktion wird an sauren Katalysatoren, vorzugsweise an sauren Heterogenkontakten, beispielsweise Phosphorsäure auf einem SiO₂-Träger, bei einer Eingangstemperatur von 150 bis 300°C, vorzugsweise bei 200 bis 250°C, und einer Ausgangstemperatur von 100 bis 250°C, vorzugsweise bei 130 bis 220°C durchgeführt.

Bei Einsatz von FCC-C₄-Schnitt ist damit zu rechnen, dass Propan in Mengen um 1 Gew.-%, Isobuten in Mengen um 30 bis 40 Gew.-% sowie C₅-Kohlenwasserstoffe in Mengen um 3 bis 10 Gew.-% eingeschleust werden, welche die nachfolgende Verfahrenssequenz beeinträchtigen können. Im Rahmen der Aufarbeitung des Ethers ist demzufolge die Möglichkeit einer destillativen Abtrennung der genannten Komponenten vorgesehen.

Der so erhaltene, als Raffinat II bezeichnete Reaktionsaustrag weist einen Isobuten-Restgehalt von 0,1 bis 3 Gew.-% auf.

Bei größeren Mengen an Isobuten im Austrag, wie beispielsweise bei Einsatz von FCC-C₄-Fraktionen oder bei der Abtrennung von Isobuten durch sauerkatalysierte Polymerisation zu Polyisobuten (Teilumsatz), kann der verbleibende Raffinatstrom gemäß einer Ausführungsform der Erfindung vor der Weiterverarbeitung destillativ aufbereitet werden.

### Reinigung des Raffinat II-Stroms an Adsorbermaterialien

Der nach der Veretherung/Polymerisation (bzw. Destillation) erhaltene Raffinat II-Strom wird an mindestens einem guard bed, bestehend aus hochoberflächigen Aluminiumoxiden, Kieselgelen, Alumosilikaten oder Molsieben, gereinigt. Das Schutzbett dient hierbei zum Trocknen des C₄-Stroms sowie zur Entfernung von Substanzen, welche als Katalysatorgift im nachfolgenden Metatheseschritt wirken können. Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die so gewählt sind, dass sämtliche Komponenten in der flüssigen Phase vorliegen. Gegebenenfalls wird der Reinigungsschritt zur Feed-Vorwärmung für den nachfolgenden Metatheseschritt eingesetzt.

Der verbleibende Raffinat II-Strom ist annähernd frei von Wasser, Oxygenaten, organischen Chloriden und Schwefelverbindungen.

Bei Durchführung des Veretherungsschritts mit Methanol zur Herstellung von MTBE kann es aufgrund der Bildung von Dimethylether als Nebenkomponente erforderlich sein, mehrere Reinigungsschritte zu kombinieren bzw. hintereinander zu schalten.

Als Metathesekatalysatoren werden literaturbekannte heterogene Rhenium-Katalysatoren, wie Re₂O₇ auf γ-Al₂O₃ oder auf Mischträgern, wie z.B. SiO₂/Al₂O₃, B₂O₃/SiO₂/Al₂O₃ oder Fe₂O₃/Al₂O₃ mit unterschiedlichem Metallgehalt bevorzugt. Der Rheniumoxid-Gehalt beträgt unabhängig vom gewählten Träger zwischen 1 und 20 %, vorzugsweise zwischen 3 und 10 %.

Die Katalysatoren werden frisch calziniert eingesetzt und bedürfen keiner weiteren Aktivierung (z.B. durch Alkylierungsmittel). Desaktivierter Katalysator kann durch Abbrennen von Coke-Rückständen bei Temperaturen oberhalb von 400°C im Luftstrom und Abkühlung unter Inertgas-Atmosphäre mehrfach regeneriert werden.

Ein Vergleich der Heterogenkontakte untereinander zeigt, dass Re₂O₇/Al₂O₃ bereits unter sehr milden Reaktionsbedingungen (T = 20 bis 80°C) aktiv ist, während MO₃/SiO₂ (M = Mo, W) erst bei Temperaturen oberhalb von 100 bis 150°C Aktivität entwickelt und demzufolge als Nebenreaktionen C=C-Doppelbindungsisomerisierung auftreten kann.

Ferner sind zu nennen:
- WO₃/SiO₂, präpariert aus (C₅H₄)W(CO)₃Cl und SiO₂ in J. Mol Catal. **1995,** 95, 75-83;
- 3-Komponenten-System, bestehend aus [Mo(NO)₂(OR)₂]n, SnEt₄ und AlCl₃ in J. Mol. Catal. **1991,** 64, 171-178 und J. Mol. Catal **1989,** 57, 207-220;
- Nitridomolybdän (VI)-Komplexe aus hochaktive Präkatalysatoren in J. Organomet. Chem. **1982,** 229, C₁₉-C₂₃;
- heterogene SiO₂-geträgert MoO₃ und WO₃-Katalysatore in J. Chem. Soc., Faraday Trans. / **1982,** 78, 2583-2592;
- geträgerte Mo-Katalysatoren in J. Chem. Soc., Faraday Trans. / **1981,** 77, 1763-1777;
- aktive Wolfram-Katalysatorvorstufe in J. Am. Chem. Soc. **1980,** 102(21), 6572-6574;
- Acetonitril(pentacarbonyl)wolfram in J. Catal. **1975,** 38, 482-484;
- Trichloro(nitrosyl)molybdän(II) als Katalysator-Vorstufe in Z. Chem. **1974,** 14, 284-285;
- W(CO)₅PPH₃/EtAlCl₂ in J. Catal. **1974,** 34, 196-202;
- WCl₆/n-BuLi in J. Catal **1973,** 28, 300-303;
- WCl₆/n-BuLi in J. Catal. **1972,** 26, 455-458;

FR 2 726 563: O₃ReO[Al(OR)(L)xO]nReO₃ mit R = C₁-C₄₀-Kohlenwasserstoff, n = 1-10, x = 0 oder 1 und L = Solvens,
EP-A-191 0 675, EP-A-129 0 474, BE 899897: Katalysatorsysteme aus Wolfram, 2-substituierten Phenolatresten und 4 anderen Liganden, u.a. einer Halogen-, Alkyl- bzw. Carbengruppe.
FR 2 499 083: Katalysatorsystem aus einem Wolfram-, Molybdän- oder Rhenium-Oxo-Übergangsmetallkomplex mit einer Lewissäure.
US 4,060,468: Katalysatorsystem aus einem Wolframsalz, einer sauerstoffhaltigen aromatischen Verbindung, z.B. 2,6-Dichlorphenol und wahlweise molekularem Sauerstoff.
BE 776,564: Katalysatorsystem aus einem Übergangsmetallsalz, einer metallorganischen Verbindung und einem Amin.

Für die Verbesserung der Cyclusdauer der eingesetzten Katalysatoren, vor allem der geträgerten Katalysatoren, empfiehlt sich der Einsatz einer Feed-Reinigung an Adsorberbetten (guard beds). Das Schutzbett dient hierbei zum Trocknen des C₄-Stroms sowie zur Entfernung von Substanzen, welches als Katalysatorgift im nachfolgenden Metatheseschritt wirken können. Die bevorzugten Adsorbermaterialien sind Selexsorb CD und CDO sowie 3Å- und NaX-Molsiebe (13X). Die Reinigung erfolgt in Trockentürmen bei Temperaturen und Drucken, die bevorzugt so gewählt sind, dass sämtliche Komponenten in der flüssigen Phase vorliegen. Gegebenenfalls wird der Reinigungsschritt zur Feed-Vorwärmung für den nachfolgenden Metatheseschritt eingesetzt. Es kann von Vorteil sein, mehrere Reinigungsschritte miteinander zu kombinieren bzw. hintereinander zu schalten.

Druck und Temperatur im Metatheseschritt sind so gewählt, dass sämtliche Reaktionspartner in der flüssigen Phase vorliegen (üblicherweise T = 0 bis 150°C, bevorzugt 20 bis 80°C; p = 2 bis 200 bar). Alternativ kann es aber von Vorteil sein, insbesondere bei Feedströmen mit höherem Isobutengehalt, die Umsetzung in der Gasphase durchzuführen und/oder einen Katalysator einzusetzen, der über eine geringere Acidität verfügt.

In der Regel ist die Umsetzung nach 1 s bis 1 h, vorzugsweise nach 30 s bis 30 min beendet. Sie kann kontinuierlich oder diskontinuierlich in Reaktoren, wie Druckgasgefäßen, Strömungsrohren oder Reaktivdestillationsvorrichtungen durchgeführt werden, wobei Strömungsrohre bevorzugt werden.

### Stufe b)

In Stufe b) wird das in Stufe a) erhaltene 2-Penten und/oder 3-Hexen in Gegenwart eines Dimerisierungskatalysators zu einem C₁₀₋₁₂-Olefin-Gemisch dimerisiert.

Die erhaltenen erfindungsgemäßen Dimer-Olefingemische weisen vorzugsweise einen mittleren Verzweigungsgrad im Bereich von 1 bis 2,5, besonders bevorzugt 1 bis 2,0, insbesondere 1 bis 1,5 und speziell 1 bis 1,2 auf. Als Verzweigungsgrad eines reinen Olefins ist dabei die Zahl der Kohlenstoffatome, die mit drei Kohlenstoffatomen verknüpft sind, plus zwei mal die Zahl der Kohlenstoffatome, die mit 4 Kohlenstoffatomen verknüpft sind, definiert. Der Verzweigungsgrad eines Rein-Olefins ist dabei leicht nach Totalhydrierung zum Alkan via ¹H-NMR über die Integration der Signale der Methylgruppen relativ zu den Methylen- und Methinprotonen messbar.

Bei Mischungen von Olefinen werden die Verzweigungsgrade mit den Molprozenten gewichtet, und so wird ein mittlerer Verzweigungsgrad errechnet.

Die molaren Anteile bestimmt man dabei optimalerweise mittels Gaschromatographie.

Die Art der Verzweigungen im Olefin ist dabei bevorzugt so gestaltet, dass nach Hydrierung weniger als 10%, bevorzugt weniger als 5%, besonders bevorzugt weniger als 1% Alkane erhalten werden, die nicht zu den Methyl-, Dimethyl-, Ethylmethyl- und Diethylalkanen zählen. Dies bedeutet, dass die Verzweigungen nur Methyl- und Ethyl-Verzweigungen sind.

Gemäß einer besonders bevorzugten Ausführungsform der Erfindung wird die Dimerisierung so durchgeführt, dass die Katalyse direkt die gewünschte vorteilhafte Zusammensetzung in Bezug auf die Verzweigungsstrukturen liefert.

Gemäß einer weiteren Ausführungsform der Erfindung werden die erhaltenen C₁₀₋₁₂-Olefine abgetrennt und 5 bis 30 Gew.-%, vorzugsweise 5 bis 20 Gew.-%, insbesondere bis 10 bis 20 Gew.-%, bezogen auf die abgetrennten C₁₀₋₁₂-Olefine, an Leichtsieder-Bestandteilen der C₁₀₋₁₂-Olefine werden abgetrennt. Als Leichtsieder-Bestandteile wird der Anteil des C₁₀₋₁₂-Olefin-Gemischs bezeichnet, der bei einer Destillation zuerst übergeht bzw. den niedrigsten Siedepunkt hat. Der genannte Gewichtsanteil entspricht somit dem Anteil, der bei einer Destillation zunächst übergeht und damit abgetrennt werden kann. Die Abtrennung kann aber auch über beliebige andere geeignete Verfahren erfolgen. Insbesondere wird eine fraktionierte Destillation durchgeführt. Durch die erfindungsgemäß durchgeführte Abtrennung werden die mehrfach verzweigten Olefine teilweise oder vorzugsweise ganz vom C₁₀₋₁₂-Olefin-Gemisch abgetrennt. Die Abtrennung kann auch so durchgeführt werden, dass mindestens 80%, vorzugsweise mindestens 90%, insbesondere mindestens 95% der zweifach oder mehrfach verzweigten Olefine abgetrennt werden. Im C₁₀₋₁₂-Olefin-Gemisch am Ende der Stufe b) verbleiben damit die linearen und einfach verzweigten Olefine und gegebenenfalls geringere Anteile mehrfach verzweigter Olefine. Dem Fachmann sind geeignete Abtrennungsverfahren und Analyseverfahren zur Bestimmung des Gehaltes an mehrfach verzweigten Olefinen bekannt.

Die genannten Ausführungsformen können mit einem Zusatz von linearen Olefinen in Stufe c), linearen Alkylbenzolen in Stufe d), linearen Alkylarylsulfonaten in Stufe e) oder Kombinationen davon kombiniert werden. Es kann jedoch auch auf einen Zusatz derartiger linearer Verbindungen verzichtet werden.

Werden in den Stufen c), d) und/oder e) lineare Verbindungen zugesetzt, so kann gemäß einer Ausführungsform auf die Abtrennung von Leichtsieder-Bestandteilen in Stufe b) verzichtet werden.

Im Dimerisierungsgemisch können < 30, bevorzugt < 10 Gew.-% Alkane und < 5 Gew.-% nicht - C₁₀₋₁₂-Olefine enthalten sein.

Vorzugsweise werden für die Dimerisierung die in dem Metatheseprodukt enthaltenen internen, linearen Pentene und Hexene eingesetzt. Besonders bevorzugt ist der Einsatz von 3-Hexen.

Die Dimerisierung kann homogenkatalysiert oder heterogenkatalysiert durchgeführt werden. Die homogenkatalysierte Dimerisierung kann in Bezug auf die Verzweigungsstrukturen in weiten Grenzen variiert werden. Neben Nickel-Systemen können beispielsweise Ti-, Zr-, Cr- oder Fe-Systeme eingesetzt werden, die über weitere Cokatalysatoren und Liganden gezielt modifiziert werden können.

Besonders bevorzugt wird die homogenkatalysierte Dimerisierung in Abwesenheit von Übergangsmetallen mit Aluminiumalkylen AlR₃ katalysiert. Während diese α-Olefine bei sehr milden Bedingungen selektiv zu Vinylidenen umsetzen, gelingt bei drastischeren Bedingungen auch die entsprechende Umsetzung von internen Olefinen. Auch hier werden Dimere mit hohem Vinyliden-Anteil gebildet. Der Anteil zweifach und dreifach verzweigter Isomere ist äußerst niedrig.

Die AlR₃-katalysierte Dimerisierung wird vorzugsweise bei Temperaturen im Bereich von 150 bis 300°C, besonders bevorzugt 180 bis 240°C, insbesondere 210 bis 230°C durchgeführt, der Katalysator wird vorzugsweise destillativ über Sumpf abgetrennt und in die Katalyse rückgeführt.

Zur heterogenen Katalyse werden zweckmäßigerweise Kombinationen von Oxiden von Metallen der VIII. Nebengruppe mit Aluminiumoxid auf Trägermaterialien aus Silizium-und Titanoxiden wie sie beispielsweise aus der DE-A-43 39 713 bekannt sind, eingesetzt. Der heterogene Katalysator kann im Festbett - dann vorzugsweise in grobkörniger Form als 1 bis 1,5 mm-Splitt - oder suspendiert (Partikelgröße 0.05 bis 0,5 mm) eingesetzt werden. Die Dimerisierung wird bei heterogener Durchführung zweckmäßigerweise bei Temperaturen von 80 bis 200°C, vorzugsweise von 100 bis 180°C, unter dem bei der Reaktionstemperatur herrschenden Druck, gegebenenfalls auch unter einem Schutzgasüberdruck, im geschlossenen System ausgeführt. Zur Erzielung optimaler Umsätze wird das Reaktionsgemisch mehrfach im Kreis geführt, wobei kontinuierlich ein bestimmter Anteil des zirkulierenden Produkts ausgeschleust und durch Ausgangsmaterial ersetzt wird.

Bei der erfindungsgemäßen Dimerisierung werden Mischungen einfach ungesättigter Kohlenwasserstoffe erhalten, deren Komponenten überwiegend die doppelte Kettenlänge haben wie die Ausgangs-Olefine.

Bei erfindungsgemäß hergestellten C₁₂-Olefingemischen trägt die Hauptkette an den Verzweigungspunkten vorzugsweise Methyl- oder Ethylgruppen.

Die nach dem vorstehenden Verfahren (vgl. WO 00/39058) erhältlichen Olefingemische stellen wertvolle Zwischenprodukte insbesondere für die im Folgenden beschriebene Herstellung von verzweigten Alkylaromaten zur Herstellung von Tensiden dar.

### Stufe c)

In Stufe c) wird das in Stufe b) erhaltene C₁₀₋₁₂-Olefin-Gemisch mit einem aromatischen Kohlenwasserstoff in Gegenwart eines Alkylierungskatalysators unter Bildung von alkylaromatischen Verbindungen umgesetzt.

Das in Stufe c) eingesetzte C₁₀₋₁₂-Olefin-Gemisch weist eine optimale Struktur/Linearität auf. Dies bedeutet, dass der Verzweigungsgrad und die Art der Verzweigung optimal gewählt sind, um in Stufe c) vorteilhafte alkylaromatische Verbindungen zu erhalten. Die Einstellung des in Stufe c) optimal einzusetzenden C₁₀₋₁₂-Olefin-Gemisches kann durch Zumischen linearer Olefine erfolgen. Vorzugsweise werden jedoch höher verzweigte Olefine abgetrennt anstelle eines Zumischens linearer Olefine. Besonders bevorzugt wird bei der Dimerisierung ein geeigneter Katalysator mit einer geeigneten Verfahrensweise kombiniert, um zum optimalen C₁₀₋₁₂-Olefin-Gemisch zu gelangen. Bei dieser Verfahrensweise werden bei der Alkylierung direkt die gewünschten Strukturen erhalten. Man kann in diesem Fall auf das Zumischen linearer Olefine und das Abtrennen höher verzweigter Olefine verzichten. Es sind auch Kombinationen der beschriebenen Verfahrensweisen möglich.

Sofern in Stufe b) eine Leichtsieder-Abtrennung durchgeführt wird, können wahlweise in Stufe c) 0 bis 60 Gew.-%, vorzugsweise 0 bis 50 Gew.-%, insbesondere 0 bis 30 Gew.-%, bezogen auf die in Stufe b) erhaltenen C₁₀₋₁₂-Olefin-Gemische, an linearen Olefinen zugesetzt werden. Sofern lineare Olefine zugesetzt werden, beträgt deren Menge mindestens 1 Gew.-%, vorzugsweise mindestens 5 Gew.-%, insbesondere mindestens 10 Gew.-%.

Sofern gemäß der zweiten Ausführungsform der Erfindung in Stufe b) keine Leichtsieder-Abtrennung durchgeführt wird, werden in mindestens einer der Stufen c), d) und e) 5 bis 60 Gew.-%, jeweils bezogen auf die in der vorherigen Stufe erhaltenen Gemische, der linearen Verbindungen zugesetzt. Dies bedeutet, dass in Stufe c) zusätzlich lineare Olefine zugesetzt werden und/oder in Stufe d) zusätzlich lineare Alkylbenzole zugesetzt werden und/oder in Stufe e) zusätzlich lineare Alkylarylsulfonate zugesetzt werden. Es können damit in jeder der Stufen c), d) und e), wie auch in einzelnen oder zweien der Stufen lineare Verbindungen zugesetzt werden. In Stufe c) können so 5 bis 60 Gew.-%, vorzugsweise 10 bis 50 Gew.-%, insbesondere 10 bis 30 Gew.-%, bezogen auf die in Stufe b) erhaltenen C₁₀₋₁₂-Olefin-Gemische, an linearen Olefinen zugesetzt werden.

Auf die Stufen c), d) und e) insgesamt bezogen werden vorzugsweise höchstens 60 Gew.-%, besonders bevorzugt höchstens 40 Gew.-%, insbesondere höchstens 30 Gew.-% der linearen Verbindungen zugesetzt. Wird diese Höchstmenge bereits durch den Zusatz in einer der Stufen erreicht, wird in den anderen Stufen auf einen Zusatz linearer Verbindungen verzichtet.

Durch den Zusatz der linearen Verbindungen kann das Eigenschaftsprofil der Alkylarylsulfonate über die vorteilhafte Synthesesequenz hinaus an das jeweilige gewünschte Anwendungsgebiet und Anforderungsprofil angepaßt werden.

Die jeweils genannten Untergrenzen können mit den jeweils genannten Obergrenzen zu erfindungsgemäß möglichen Bereichen kombiniert werden.

Dabei wird vorzugsweise ein Alkylierungskatalysator eingesetzt, der zu alkylaromatischen Verbindungen führt, die im Alkylrest ein bis drei Kohlenstoffatome mit einem H/C-Index von 1 aufweisen.

Die Alkylierung kann im Prinzip in Gegenwart beliebiger Alkylierungskatalysatoren durchgeführt werden.

Obwohl AlCl₃ und HF im Prinzip einsetzbar sind, bieten heterogene bzw. formselektive Katalysatoren Vorteile. Aus Gründen der Anlagensicherheit und des Umweltschutzes werden heute Feststoffkatalysatoren bevorzugt, dazu zählen zum Beispiel der im DETAL-Prozeß eingesetzte fluorierte Si/Al-Katalysator, eine Reihe von formselektiven Katalysatoren bzw. geträgerte Metalloxidkatalysatoren, sowie Schichtsilikate und Tone.

Bei der Auswahl des Katalysators ist ungeachtet des großen Einflusses des eingesetzten Feedstocks die Minimierung von durch den Katalysator gebildeten Verbindungen wichtig, die dadurch gekennzeichnet sind, dass sie im Alkylrest C-Atome mit einem H/G-Index von 0 beinhalten. Des weiteren sollen Verbindungen gebildet werden, die im Mittel im Alkylrest 1 bis 3 C-Atome mit einem H/C-Index von 1 aufweisen. Dies kann insbesondere durch die Auswahl geeigneter - Katalysatoren erreicht werden, die einerseits durch ihre Geometrie die Bildung der unerwünschten Produkte unterdrücken und andererseits aber eine ausreichende Reaktionsgeschwindigkeit zulassen.

Die erfindungsgemäßen alkylaromatischen Verbindungen weisen einen charakteristischen Anteil von primären, sekundären, tertiären und quartären Kohlenstoffatomen im Alkylrest (Seitenkette) auf. Dieser spiegelt sich wider in der Anzahl von Kohlenstoffatomen im Alkylrest mit einem H/C-Index von 0 bis 3. Der H/C-Index definiert dabei die Anzahl der Protonen pro Kohlenstoffatom im Alkylrest. Vorzugsweise weisen die erfindungsgemäßen Gemische alkylaromatischer Verbindungen nur einen geringen Anteil an C-Atomen im Alkylrest mit einem H/C-Indes von 0 auf. Vorzugsweise ist der Anteil an C-Atomen im Alkylrest mit einem H/C-Index von 0 im Mittel aller Verbindungen < 15%, besonders bevorzugt < 10%. Der Anteil an Kohlenstoffatomen im Alkylrest mit einem H/C-Index von 0, die gleichzeitig an den Aromaten gebunden sind, beträgt ≥ 80%, bevorzugt ≥ 90%, besonders bevorzugt ≥ 95% aller Kohlenstoffatome im Alkylrest mit einem H/C-Index von 0.

Vorzugsweise weisen die erfindungsgemäßen Gemische alkylaromatischer Verbindungen im Mittel 1 bis 3, bevorzugt 1 bis 2,5, besonders bevorzugt 1 bis 2 Kohlenstoffatome in der Seitenkette (d.h. ohne die aromatischen C-Atome zu zählen) mit einem H/C-Index von 1 auf. Der Anteil an Verbindungen mit drei Kohlenstoffatomen dieses Typs liegt bevorzugt bei < 30%, besonders bevorzugt < 20%, insbesondere < 10%.

Eine Steuerung des Anteils der Kohlenstoffatome, die einen bestimmten H/C-Index aufweisen, kann durch geeignete Wahl des eingesetzten Katalysators erfolgen. Bevorzugt eingesetzte Katalysatoren, mit denen vorteilhafte H/C-Verteilungen erzielt werden, sind Mordenit, β-Zeolith, L-Zeolith, MCM-58, MCM-68 und Faujasit. Insbesondere bevorzugt sind Mordenit und Faujasit.

Bei der Auswahl der Katalysatoren ist darüber hinaus auf deren Neigung hinsichtlich Deaktivierung zu achten. Eindimensionale Porensysteme weisen meistens den Nachteil einer raschen Verstopfung der Poren durch Abbau- bzw. Aufbauprodukte aus dem Prozess auf. Katalysatoren mit mehrdimensionalen Porensystemen sind daher zu bevorzugen.

Die eingesetzten Katalysatoren können natürlichen oder synthetischen Ursprungs sein, deren Eigenschaften sind durch literaturbekannte Methoden (z.B. Ionenaustausch, Steaming, Blockierung azider Zentren, Auswaschen von Extra-Gitter-Spezies, etc.) in gewissem Umfang einstellbar. Wichtig für die vorliegende Erfindung ist, dass die Katalysatoren zumindest zum Teil sauren Charakter aufweisen.

Je nach Anwendungsart liegen die Katalysatoren entweder als Pulver oder als Formkörper vor. Die Verbindungen der Matrizes der Formkörper gewährleisten ausreichende mechanische Stabilität jedoch ist ein freier Zugang der Moleküle zu den aktiven Bestandteilen der Katalysatoren durch ausreichende Porosität der Matrices zu gewährleisten. Die Herstellung solcher Formkörper ist literaturbekannt und wird nach dem Stand der Technik ausgeführt.

### Bevorzugte Reaktionsdurchführung

Die Alkylierung wird derart durchgeführt, dass man den Aromaten (das Aromatengemisch) und das Olefin(gemisch) in einer geeigneten Reaktionszone durch Inkontaktbringen mit dem Katalysator reagieren lässt, nach der Reaktion das Reaktionsgemisch aufarbeitet und so die Wertprodukte gewinnt.

Geeignete Reaktionszonen stellen z.B. Rohrreaktoren oder Rührkessel dar. Liegt der Katalysator in fester Form vor, so kann er entweder als Aufschlämmung (Slurry), als Festbett oder als Wirbelbett eingesetzt werden. Auch die Ausführung als katalytische Destillation ist möglich.

Die Reaktionspartner liegen entweder in flüssigem und/oder in gasförmigem Zustand vor.

Die Reaktionstemperatur wird so gewählt, dass auf der einen Seite möglichst vollständiger Umsatz des Olefins stattfindet und auf der anderen Seite möglichst wenig Nebenprodukte entstehen. Die Wahl der Temperaturführung hängt außerdem entscheidend vom gewählten Katalysator ab. Reaktionstemperaturen zwischen 50°C und 500°C (bevorzugt 80 bis 350°C, besonders bevorzugt 80 bis 250°C) sind anwendbar.

Der Druck der Reaktion richtet sich nach der gewählten Fahrweise (Reaktortyp) und beträgt zwischen 0,1 und 100 bar, die Katalysatorbelastung (WHSV) wird zwischen 0,1 und 100 gewählt. In der Regel arbeitet man bei Eigendruck (dem Dampfdruck des Systems) oder darüber.

Die Reaktionspartner können optional mit inerten Stoffen verdünnt werden. Inerte Stoffe sind bevorzugt Paraffine.

Das molare Verhältnis von Aromat:Olefin wird üblicherweise zwischen 1:1 und 100:1 (bevorzugt 2:1-20:1) eingestellt.

### Aromatische Einsatzstoffe

Möglich sind alle aromatischen Kohlenwasserstoffe der Formel Ar-R, wobei Ar einen monocyclischen oder bicyclischen aromatischen Kohlenwasserstoff-Rest darstellt und R aus H, C₁₋₅ bevorzugt C₁₋₃-Alkyl, OH, OR etc., bevorzugt H oder C₁₋₃-Alkyl ausgewählt ist. Bevorzugt sind Benzol und Toluol.

### Stufe d)

In Stufe d) werden die in Stufe c) erhaltenen alkylaromatischen Verbindungen sulfoniert und zu Alkylarylsulfonaten neutralisiert.

Die Alkylaryle werden durch
1) Sulfonierung (z.B. mit SO₃, Oleum, Chlorsulfonsäure, etc., bevorzugt mit SO₃) und
2) Neutralisation (z.B. mit Na-, K-, NH₄-, Mg-Verbindungen, bevorzugt mit Na-Verbindungen)
zu Alkylarylsulfonaten umgesetzt. Sulfonierung und Neutralisation sind in der Literatur hinreichend beschrieben und werden nach dem Stand der Technik ausgeführt. Die Sulfonierung wird bevorzugt in einem Fallfilmreaktor ausgeführt, kann aber auch in einem Rührkessel erfolgen. Die Sulfonierung mit SO₃ ist der Sulfonierung mit Oleum vorzuziehen.

### Mischungen

Die nach oben beschriebenen Verfahren hergestellten Verbindungen werden entweder als solche weiterverarbeitet, oder vorher mit linearen Alkylarylen gemischt und dann der Weiterverarbeitung zugeführt. Um diesen Prozess zu vereinfachen, kann es auch sinnvoll sein, die Rohstoffe, die zur Herstellung der oben genannten anderen Alkylaryle verwendet werden, direkt mit den Rohstoffen des vorliegenden Verfahrens zu mischen und dann das erfindungsgemäße Verfahren durchzuführen. So ist z.B., wie beschrieben, die Mischung von leicht verzweigten Olefinströmen aus dem erfindungsgemäßen Verfahren mit linearen Olefinen sinnvoll. Auch Mischungen der Alkylarylsulfonsäuren bzw. der Alkylarylsulfonate sind anwendbar. Die Mischungen werden immer in Hinblick auf die Optimierung der Produktqualität der aus dem Alkylaryl gefertigten Tenside vorgenommen.

In Stufe d) können vor der Sulfonierung zusätzlich lineare Alkylbenzole zugesetzt werden. Ihre Menge beträgt 0 bis 60 Gew.-%, vorzugsweise 0 bis 50 Gew.-%, insbesondere 0 bis 30 Gew.-%. Sofern in Stufe b) keine Leichtsieder-Abtrennung durchgeführt wird, und in den Stufen c) und e) kein Zusatz linearer Verbindungen erfolgt, beträgt die Mindestmenge 5 Gew.-%, vorzugsweise 10 Gew.-%. Auf die vorstehenden Ausführungen zur Gesamtmenge der zugesetzten linearen Verbindungen wird verwiesen. In den linearen Alkylbenzolen entspricht die Kettenlänge der Alkylreste vorzugsweise der Kettenlänge der Alkylreste, wie sie aus Stufe c) in den alkylaromatischen Verbindungen erhalten wird. Zu (C₁₀-Alkyl)-benzolen werden vorzugsweise lineare (C₁₀-Alkyl)-benzole zugesetzt, entsprechend zu (C₁₂-Alkyl)-benzolen lineare (C₁₂-Alkyl)-benzole.

Einen beispielhaften Überblick über Alkylierung, Sulfonierung, Neutralisation gibt z.B. "Alkylarylsulfonates: History, Manufacture, Analysis and Environmental Properties" in Surf. Sci. Ser. 56 (1996) Kapitel 2, Marcel Dekker, New York und darin enthaltene Referenzen.

### Stufe e)

In Stufe e) können die in Stufe d) erhaltenen Alkylarylsulfonate zusätzlich mit linearen Alkylarylsulfonaten abgemischt werden.

In Stufe e) werden vorzugsweise 0 bis 60 Gew.-%, besonders bevorzugt 0 bis 50 Gew.-%, insbesondere 0 bis 30 Gew.-% lineare Alkylarylsulfonate zugemischt. Sofern in Stufe b) keine Abtrennung von Leichtsieder-Bestandteilen stattfindet und in den Stufen c) und d) kein Zusatz linearer Verbindungen erfolgt, beträgt die Mindestmenge vorzugsweise 5 Gew.-%, vorzugsweise mindestens 10 Gew.-%. Auf die vorstehend angegebenen bevorzugten Gesamtmengen beim Zusatz linearer Verbindungen wird verwiesen.

Sämtliche Gewichtsangeben beziehen sich jeweils auf die in der vorhergehenden Stufe erhaltenen Gemische.

Die Erfindung betrifft auch Alkylarylsulfonate, die nach einem wie vorstehend beschriebenen Verfahren erhältlich sind.

Die erfindungsgemäßen Alkylarylsulfonate werden vorzugsweise als Tenside, insbesondere in Wasch- und Reinigungsmitteln eingesetzt. Die Erfindung betrifft auch ein Wasch- und Reinigungsmittel, enthaltend neben üblichen Inhaltsstoffen Alkylarylsulfonate, wie sie vorstehend beschrieben sind.

Nicht ausschließliche Beispiele üblicher Inhaltsstoffe der erfindungsgemäßen Wasch- und Reinigungsmittel sind z.B. in WO 02/44114 und WO 02/14266 aufgeführt.

Die Erfindung wird anhand der nachstehenden Beispiele näher erläutert.

### Beispiel 1

Eine butadienfreie C₄-Fraktion mit einem Gesamtbutengehalt von 84,2 Gew.-% sowie einem Molverhältnis 1-Buten zu 2-Butene von 1 zu 1,06 wird bei 40°C und 10 bar kontinuierlich über einen mit Re₂O₇/Al₂O₃-Heterogenkontakt bestückten Rohrreaktor geleitet. Die Katalysator-Belastung beträgt im Beispiel 4500 kg/m²h. Der Reaktionsaustrag wird destillativ getrennt und enthält folgende Komponenten (Angaben in Massenprozent):
Ethen 1,15 %; Propen 18,9 %, Butane 15,8 %, 2-Butene 19,7 %, 1-Buten 13,3 %, i-Buten 1,0 %, 2-Penten 19,4 %, Methylbutene 0,45 %, 3-Hexen 10,3 %.
   2-Penten und 3-Hexen werden aus dem Produkt destillativ in Reinheiten > 99 Gew.-% gewonnen.

### Beispiel 2

Kontinuierliche Dimerisierung von 3-Hexen im Festbettverfahren

| | |
|---|---|
| Katalysator: | 50 % NiO, 34 % SiO₂, 13 % TiO₂, 3 % Al₂O₃ (gemäß DE 43 39 713) eingesetzt als 1-1,5 mm Splitt (100 ml), 24 h bei 160°C in N₂ konditioniert |
| Reaktor: | isotherm, 16 mm-∅-Reaktor |
| WHSV: | 0,25 kg/l.h |
| Druck: | 20 bis 25 bar |
| Temperatur: | 100 bis 160°C |

| | | | | | | | | |
|---|---|---|---|---|---|---|---|---|
| Temperatur | | 100 | 120 | 140 | 160 | 160 | | |
| (°C) | | | | | | | | |
| Druck | **Feed-** | 20 | 20 | 20 | 25 | 25 | Sammel | **C₁₂-** |
| (bar) | **stock** | | | | | | - | **Destillat** |
| Betriebsstunden | | 12 | 19 | 36 | 60 | 107 | Produkt | |
| Flüssiganfall | | 24 | 27 | 27 | 28 | 27 | | |
| (g/h) | | | | | | | | |
| Zusammensetzung | | | | | | | | |
| (Gew.-%) | | | | | | | | |
| C₆ | 99,9 | 68,5 | 52,7 | 43,6 | 57,0 | 73,2 | n.e. | 0,1 |
| C₇-C₁₁ | 0,1 | 0,2 | 0,2 | 0,3 | 0,2 | 0,2 | | - |
| C₁₂ | | 25,9 | 38,6 | 44,0 | 35,6 | 23,6 | | 99,9 |
| C₁₃+ | | 5,4 | 8,5 | 12,1 | 7,2 | 3,0 | | - |
| | | | | | | | | |
| Umsatz | | 31,4 | 47,2 | 56,4 | 42,9 | 26,7 | | |
| C12-Selektivität | | 82,5 | 81,8 | 78,2 | 83,0 | 88,4 | | |
| (Gew.-%) | | | | | | | | |
| S-Gehalt im | < 1 | n.e. | n.e. | n.e. | n.e. | n.e. | n.e. | n.e. |
| Flüssiganfall | | | | | | | | |
| (ppm) | | | | | | | | |

Das Sammel-Produkt wurde bis zu einer C₁₂-Reinheit von 99,9 Gew.-% aufdestilliert.

### Beispiel 3

2-Penten aus der Raffinat II-Metathese wurde analog zu Beispiel 2 kontinuierlich an einem Ni-Heterogenkatalysator dimerisiert. Durch fraktionierte Destillation des Produktes wurde eine Decenfraktion mit einer Reinheit von 99,5 % erhalten.

### Beispiel 4

Eine Mischung von 2-Penten und 3-Hexen aus der Raffinat II-Metathese wurde analog zu Beispiel 2 und Beispiel 3 kontinuierlich dimerisiert. Durch fraktionierte Destillation des Produktes wurde eine Decen-/Undecen-/Dodecenfraktion mit einer Reinheit von 99,5 % erhalten.

### Beispiel 5

100 g 3-Hexen werden mit 3 g Triethylaluminium zur Reaktion gebracht. Nach 22 Stunden bei einer Temperatur von 220°C wird die Umsetzung beendet.

Im erhaltenen Produktgemisch beträgt das Molverhältnis von Dimer zu Trimer 58. Der Anteil an 2-Butyl-1-octen beträgt 69%. Der Verzweigungsgrad beträgt 1,03. Der Anteil an 2- und 3-fach verzweigten Isomeren beträgt 2%.

### Beispiel 6

Ein ein einem Umluftofen befindlicher Rohrreaktor wurde mit 32 g Katalysatorsplitt (60% H-Mordenit mit SiO₂ : Al₂O₃ = 24,5 - verformt mit 40% Pural^{®} SB von Condea) der Korngröße 0,7 - 1 mm gefüllt und 6 Stunden bei 500°C aktiviert. Dann wurde abgekühlt mit einem Feed aus Benzol: Dodecen aus Beispiel 5 (10:1 molar) geflutet, mit 0,62 g/g_{Kat}h belastet und ein 10-fach höherer Umlaufstrom eingestellt. Schließlich wurde der Reaktor auf 180°C (einphasig flüssig, 30 bar hydraulischer Druck) erhitzt und zeitaufgelöst der Gehalt an Edukten und Produkten im Auslaufstrom mittels GC detektiert. Das erhaltene C₁₈-Alkylarylgemisch wurde destillativ gereinigt und mittels Gaschromatographie-Massenspektrometrie Kopplung und ¹H/¹³C-NMR analysiert.

## Patentansprüche

1. Verfahren zur Herstellung von Alkylarylsulfonaten durch
a) Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen,
b) Dimerisierung des in Stufe a) erhaltenen 2-Pentens und/oder 3-Hexens in Gegenwart eines Dimerisierungskatalysators zu einem C₁₀₋₁₂-Olefine enthaltenden Gemisch, Abtrennung der C₁₀₋₁₂-Olefine und Abtrennung von 5 bis 30 Gew.-%, bezogen auf die abgetrennten C₁₀₋₁₂-Olefine, an Leichtsieder-Bestandteilen der C₁₀₋₁₂-Olefine,
c) Umsetzung der in Stufe b) erhaltenen C₁₀₋₁₂-Olefin-Gemische mit einem aromatischen Kohlenwasserstoff in Gegenwart eines Alkylierungskatalysators zur Bildung von alkylaromatischen Verbindungen, wobei vor der Umsetzung zusätzlich 0 bis 60 Gew.-%, bezogen auf die in Stufe b) erhaltenen C₁₀₋₁₂-Olefin-Gemische, an linearen Olefinen zugesetzt werden können,
d) Sulfonierung der in Stufe c) erhaltenen alkylaromatischen Verbindungen und Neutralisation zu Alkylarylsulfonaten, wobei vor der Sulfonierung zusätzlich 0 bis 60 Gew.-%, bezogen auf die in Stufe c) erhaltenen alkylaromatischen Verbindungen, an linearen Alkylbenzolen zugesetzt werden können, sofern keine Zumischung in Stufe c) erfolgt ist,
e) gegebenenfalls Abmischen der in Stufe d) erhaltenen Alkylarylsulfonate mit 0 bis 60 Gew.-%, bezogen auf die in Stufe d) erhaltenen Alkylarylsulfonate, an linearen Alkylarylsulfonaten, sofern keine Zumischungen in Stufen c) und d) erfolgt sind.

2. Verfahren zur Herstellung von Alkylarylsulfonaten durch
a) Umsetzung eines C₄-Olefin-Gemisches an einem Metathesekatalysator zur Herstellung eines 2-Penten und/oder 3-Hexen enthaltenden Olefingemisches und gegebenenfalls Abtrennung von 2-Penten und/oder 3-Hexen,
b) Dimerisierung des in Stufe a) erhaltenen 2-Pentens und/oder 3-Hexens in Gegenwart eines Dimerisierungskatalysators zu einem C₁₀₋₁₂-Olefine enthaltenden Gemisch und gegebenenfalls Abtrennung der C₁₀₋₁₂-Olefine,
c) Umsetzung der in Stufe b) erhaltenen C₁₀₋₁₂-Olefin-Gemische mit einem aromatischen Kohlenwasserstoff in Gegenwart eines Alkylierungskatalysators zur Bildung von alkylaromatischen Verbindungen, wobei vor der Umsetzung zusätzliche lineare Olefine zugesetzt werden können,
d) Sulfonierung der in Stufe c) erhaltenen alkylaromatischen Verbindungen und Neutralisation zu Alkylarylsulfonaten, wobei vor der Sulfonierung zusätzlich lineare Alkylbenzole zugesetzt werden können,
e) gegebenenfalls Abmischen der in Stufe d) erhaltenen Alkylarylsulfonate mit linearen Alkylarylsulfonaten,
wobei in mindestens einer der Stufen c), d) und e) 5 bis 60 Gew.-%, jeweils bezogen auf die in der vorherigen Stufe erhaltenen Gemische, der linearen Verbindungen zugesetzt werden und die Summe der Zusetzungen nicht mehr als 80 Gew.-% beträgt.

3. Verfahren nach Anspruch 1 oder 2, **dadurch gekennzeichnet, dass** der Metathesekatalysator in Stufe a) ausgewählt ist aus Verbindungen eines Metalls der Gruppe VIb, VIIb der VIII. Nebengruppe des Periodensystems der Elemente.

4. Verfahren nach einem der Ansprüche 1 bis 3, **dadurch gekennzeichnet, dass** man in Stufe b) einen Dimerisierungskatalysator einsetzt, der wenigstens ein Element der VIII. Nebengruppe des Periodensystems der Elemente enthält.

5. Verfahren nach einem der Ansprüche 1 bis 4, **dadurch gekennzeichnet, dass** die in Stufe b) erhaltenen Dimer-Olefingemische einen mittleren Verzweigungsgrad im Bereich von 1 bis 2,5 aufweisen.

6. Verfahren nach Anspruch 5, **dadurch gekennzeichnet, dass** die in Stufe b) erhaltenen Dimer-Olefingemische einen mittleren Verzweigungsgrad im Bereich von 1 bis 2,0 aufweisen.

7. Verfahren nach einem der Ansprüche 1 bis 6, **dadurch gekennzeichnet, daß** in Stufe c) ein Alkylierungskatalysator eingesetzt wird, der zu alkylaromatischen Verbindungen führt, die im Alkylrest 1 bis 3 Kohlenstoffatome mit einem H/C-Index von 1 aufweisen.

## Claims

1. A process for the preparation of alkylarylsulfonates by
a) reaction of a C₄-olefin mixture over a metathesis catalyst to prepare an olefin mixture comprising 2-pentene and/or 3-hexene, and if appropriate removal of 2-pentene and/or 3-hexene,
b) dimerization of the 2-pentene and/or 3-hexene obtained in stage a) in the presence of a dimerization catalyst to give a mixture comprising C₁₀₋₁₂-olefins, removal of the C₁₀₋₁₂-olefins and removal of 5 to 30% by weight, based on the C₁₀₋₁₂-olefins removed, of low-boiling constituents of the C₁₀₋₁₂-olefins,
c) reaction of the C₁₀₋₁₂-olefin mixtures obtained in stage b) with an aromatic hydrocarbon in the presence of an alkylation catalyst to form alkyl aromatic compounds, where, prior to the reaction, 0 to 60% by weight, based on the C₁₀₋₁₂-olefin mixtures obtained in stage b), of linear olefins may additionally be added,
d) sulfonation of the alkyl aromatic compounds obtained in stage c) and neutralization to give alkylarylsulfonates, where, prior to the sulfonation, 0 to 60% by weight, based on the alkyl aromatic compounds obtained in stage c), of linear alkylbenzenes may additionally be added, if no admixing has taken place in stage c),
e) if appropriate mixing of the alkylarylsulfonates obtained in stage d) with 0 to 60% by weight, based on the alkylarylsulfonates obtained in stage d), of linear alkylarylsulfonates, if no admixing has taken place in stages c) and d).

2. A process for the preparation of alkylarylsulfonates by
a) reaction of a C₄-olefin mixture over a metathesis catalyst to prepare an olefin mixture comprising 2-pentene and/or 3-hexene and if appropriate removal of 2-pentene and/or 3-hexene,
b) dimerization of the 2-pentene and/or 3-hexene obtained in stage a) in the presence of a dimerization catalyst to give a mixture comprising C₁₀₋₁₂-olefins and if appropriate removal of the C₁₀₋₁₂-olefins,
c) reaction of the C₁₀₋₁₂-olefin mixtures obtained in stage b) with an aromatic hydrocarbon in the presence of an alkylation catalyst to form alkyl aromatic compounds, where, prior to the reaction, additional linear olefins may be added,
d) sulfonation of the alkyl aromatic compounds obtained in stage c) and neutralization to give alkylarylsulfonates, where, prior to the sulfonation, linear alkylbenzenes may additionally be added,
e) if appropriate mixing of the alkylarylsulfonates obtained in stage d) with linear alkylarylsulfonates,
where, in at least one of stages c), d) and e), 5 to 60% by weight, in each case based on the mixtures obtained in the previous stage, of the linear compounds are added and the sum of the additions is not more than 80% by weight.

3. The process according to claim 1 or 2, wherein the metathesis catalyst in stage a) is chosen from compounds of a metal of group VIb, VIIb or sub-group VIII of the Periodic Table of the Elements.

4. The process according to one of claims 1 to 3, wherein, in stage b), a dimerization catalyst is used which comprises at least one element of sub-group VIII of the Periodic Table of the Elements.

5. The process according to one of claims 1 to 4, wherein the dimer olefin mixtures obtained in stage b) have an average degree of branching in the range from 1 to 2.5.

6. The process according to claim 5, wherein the dimer olefin mixtures obtained in stage b) have an average degree of branching in the range from 1 to 2.0.

7. The process according to one of claims 1 to 6, wherein, in stage c), an alkylation catalyst is used which leads to alkyl aromatic compounds which have 1 to 3 carbon atoms with a H/C index of 1 in the alkyl radical.

## Revendications

1. Procédé de préparation d'alkylarylsulfonates par
a) réaction d'un mélange oléfinique en C₄ sur un catalyseur de métathèse afin de préparer un mélange oléfinique contenant du 2-pentène et/ou du 3-hexène et éventuellement séparation de 2-pentène et/ou de 3-hexène,
b) dimérisation du 2-pentène et/ou du 3-hexène obtenus dans l'étape a) en présence d'un catalyseur de dimérisation pour donner un mélange contenant des oléfines en C₁₀₋₁₂, séparation des oléfines en C₁₀₋₁₂ et séparation de 5 à 30 % en poids, par rapport aux oléfines en C₁₀₋₁₂ séparées, des composants à bas point d'ébullition des oléfines en C₁₀₋₁₂,
c) réaction des mélanges oléfiniques en C₁₀₋₁₂ obtenus dans l'étape b) avec un hydrocarbure aromatique en présence d'un catalyseur d'alkylation pour former des composés alkylaromatiques, un supplément de 0 à 60 % en poids, par rapport aux mélanges oléfiniques en C₁₀₋₁₂ obtenus dans l' étape b), d'oléfines linéaires pouvant être ajouté avant la réaction,
d) sulfonation des composés alkylaromatiques obtenus dans l'étape c) et neutralisation pour donner des alkylarylsulfonates, un supplément de 0 à 60 % en poids, par rapport aux composés alkylaromatiques obtenus dans l'étape c), d'alkylbenzènes linéaires pouvant être ajouté avant la sulfonation, pour autant qu'aucun ajout en mélange n'ait eu lieu dans l'étape c),
e) mélange éventuel des alkylarylsulfonates obtenus dans l'étape d) avec 0 à 60 % en poids, par rapport aux alkylarylsulfonates obtenus dans l'étape d), d'alkylarylsulfonates linéaires, pour autant qu'aucun ajout en mélange n'ait eu lieu dans les étapes c) et d).

2. Procédé de préparation d'alkylarylsulfonates par
a) réaction d'un mélange oléfinique en C4 sur un catalyseur de métathèse afin de préparer un mélange oléfinique contenant du 2-pentène et/ou du 3-hexène et éventuellement séparation de 2-pentène et/ou de 3-hexène,
b) dimérisation du 2-pentène et/ou du 3-hexène obtenus dans l'étape a) en présence d'un catalyseur de dimérisation pour donner un mélange contenant des oléfines en C₁₀₋₁₂ et séparation éventuelle des oléfines en C₁₀₋₁₂,
c) réaction des mélanges oléfiniques en C₁₀₋₁₂ obtenus dans l'étape b) avec un hydrocarbure aromatique en présence d'un catalyseur d'alkylation pour former des composés alkylaromatiques, des oléfines linéaires supplémentaires pouvant être ajoutées avant la réaction,
d) sulfonation des composés alkylaromatiques obtenus dans l'étape c) et neutralisation pour donner des alkylarylsulfonates, des alkylbenzènes linéaires supplémentaires pouvant être ajoutés avant la sulfonation,
e) mélange éventuel des alkylarylsulfonates obtenus dans l'étape d) avec des alkylarylsulfonates linéaires,
dans lequel 5 à 60 % en poids, respectivement par rapport aux mélanges obtenus dans l'étape précédente, sont ajoutés aux composés linéaires dans au moins une des étapes c), d) et e) et la somme des adjonctions n'est pas supérieure à 80 % en poids.

3. Procédé selon la revendication 1 ou 2, **caractérisé en ce que** le catalyseur de métathèse est choisi dans l'étape a) parmi des composés d'un métal des groupes VIb, VIIb du VIII^{e} sous-groupe du système périodique des éléments.

4. Procédé selon l'une quelconque des revendications 1 à 3, **caractérisé en ce qu'**un catalyseur de dimérisation est utilisé dans l'étape b), qui contient au moins un élément du VIII^{e} sous-groupe du système périodique des éléments.

5. Procédé selon l'une quelconque des revendications 1 à 4, **caractérisé en ce que** les mélanges dimère-oléfine obtenus dans l'étape b) présentent un degré de ramification moyen dans la plage de 1 à 2,5.

6. Procédé selon la revendication 5, **caractérisé en ce que** les mélanges dimère-oléfine obtenus dans l'étape b) présentent un degré de ramification moyen dans la plage de 1 à 2,0.

7. Procédé selon l'une quelconque des revendications 1 à 6, **caractérisé en ce qu'**un catalyseur d'alkylation, qui mène à des composés alkylaromatiques présentant dans le radical alkyle 1 à 3 atomes de carbone avec un indice H/C de 1, est utilisé dans l'étape c).
